# EUROPEAN PATENT APPLICATION

(11) **EP 4 461 747 A1**
(43) Date of publication of application: **13.11.2024**
(21) Application number: 23737138.0
(22) Date of filing: 06.01.2023
(51) Int. Cl.: C07K 16/28, C07K 16/30, C07K 19/00, C12N 15/85

(54) **ANTIGEN BINDING PROTEIN TARGETING MSLN AND USE THEREOF**

(30) Priority: 06.01.2022 CN 202210011545; 07.01.2022 CN 202210018557
(71) Applicant: Oricell Therapeutics Co., Ltd., Shanghai 201203 (CN)
(72) Inventor: WANG, Huajing, Shanghai 201203 (CN); ZHONG, Zhenwei, Shanghai 201203 (CN); CHENG, Chao, Shanghai 201203 (CN); XIE, Ermin, Shanghai 201203 (CN); CHEN, Xiaorui, Shanghai 201203 (CN); HE, Xiaowen, Shanghai 201203 (CN)
(74) Representative: Weickmann & Weickmann PartmbB
(86) International application number: PCT/CN2023/070928
(87) International publication number: WO 2023/131276

(57) **Abstract**

An isolated antigen binding protein capable of targeting MSLN. The isolated antigen binding protein comprises at least one CDR in an antibody heavy chain variable region VH, and the VH comprises an amino acid sequence as set forth in any one of SEQ ID NO: 8, SEQ ID NO: 13, SEQ ID NO: 69, and SEQ ID NO: 70. Also provided are a chimeric antigen receptor comprising the antigen binding protein, and use of the antigen binding protein in treating tumors.

## Description

### Field of the Invention

The present application relates to the field of biomedicine, and particularly to an antigen binding protein targeting MSLN, a chimeric antigen receptor including the antigen binding protein, and use thereof.

### Background of the Invention

Mesothelin (MSLN), a glycoprotein on the cell surface, is anchored on the cell membrane via glycosyl phosphatidyl inositol. The mesothelin gene encodes a precursor protein of 69 kDa, which is hydrolyzed by furin (a paired alkaline amino acid protease)-like converting enzyme into two chains, with a membrane-bound protein of about 40 KD at the C-terminal as the mature mesothelin, and a fragment of about 30 KD at the N-terminal called megakaryocyte-promoting factor (MPF) being shed and released outside the cell. MPF and membrane-anchored MSLN are both N- glycosylated, of which MPF can promote the formation of megakaryocyte clones *in vitro,* and membrane-anchored MSLN can interact with MUC 16 and play an important role in the process of cell adhesion. Therefore, membrane-anchored MSLN is currently selected as the target of targeted therapies, and therefore, at present, MSLN specifically refers to the 40 KD fragment at the C-terminal of MSLN, i.e. membrane-anchored MSLN.

Mesothelin is a glycoprotein present on the cell surface of mesothelial cell lines in peritoneal, pleural, and pericardial cavities. Mesothelin is preferentially expressed (over-expressed) in mesothelioma, i.e., cancerous/tumor cells, ovarian cancer, pancreatic cancer, gastric cancer, lung cancer, and endometrial cancer. In contrast, its expression is limited in normal cells, e.g., mesothelial cells.

### Summary of the Invention

The present application provides an isolated antigen binding protein capable of specifically binding to MSLN. The present application further provides a chimeric antigen receptor including the antigen binding protein, and a cell including and/or expressing the chimeric antigen receptor. The cell has one or more of the following properties: (1) a strong amplification ability; (2) capable of killing MSLN-expressing target cells; (3) secreting cytokines under the stimulation of target cells; (4) inhibiting the growth of tumor cells.

In one aspect, the present application provides an isolated antigen binding protein, which includes at least one CDR in an antibody heavy chain variable region VH, the VH includes an amino acid sequence as set forth in any one of SEQ ID NO: 8, SEQ ID NO: 13, SEQ ID NO: 69, and SEQ ID NO: 70.

In some embodiments, the antigen binding protein includes an HCDR3, and the HCDR3 includes an amino acid sequence as set forth in any one of SEQ ID NO: 1, SEQ ID NO: 9, SEQ ID NO: 65, and SEQ ID NO: 66.

In some embodiments, the antigen binding protein includes an HCDR3, and the HCDR3 includes an amino acid sequence as set forth in any one of SEQ ID NO: 1, SEQ ID NO: 9, SEQ ID NO: 14, SEQ ID NO: 19, SEQ ID NO: 24, SEQ ID NO: 29, and SEQ ID NO: 34.

In some embodiments, the antigen binding protein includes an HCDR2, and the HCDR2 includes an amino acid sequence as set forth in SEQ ID NO: 67.

In some embodiments, the antigen binding protein includes an HCDR2, and the HCDR2 includes an amino acid sequence as set forth in any one of SEQ ID NO: 2, SEQ ID NO: 10, SEQ ID NO: 15, SEQ ID NO: 20, SEQ ID NO: 25, SEQ ID NO: 30, and SEQ ID NO: 35.

In some embodiments, the antigen binding protein includes an HCDR1, and the HCDR1 includes an amino acid sequence as set forth in SEQ ID NO: 68 (X₁X₂X₃MG, where X₁ is N, R, S, T or Y, X₂ is N or Y, and X₃ is A, N or V).

In some embodiments, the antigen binding protein includes an HCDR1, and the HCDR1 includes an amino acid sequence as set forth in any one of SEQ ID NO: 3, SEQ ID NO: 11, SEQ ID NO: 16, SEQ ID NO: 21, SEQ ID NO: 26, SEQ ID NO: 31, and SEQ ID NO: 36.

In some embodiments, the antigen binding protein includes an HCDR1, an HCDR2, and an HCDR3, and the HCDR1, HCDR2, and HCDR3 includes any one group of amino acid sequences selected from:
(1) the HCDR1 includes an amino acid sequence as set forth in SEQ ID NO: 3, the HCDR2 includes an amino acid sequence as set forth in SEQ ID NO: 2, and the HCDR3 includes an amino acid sequence as set forth in SEQ ID NO: 1;
(2) the HCDR1 includes an amino acid sequence as set forth in SEQ ID NO: 11, the HCDR2 includes an amino acid sequence as set forth in SEQ ID NO: 10, and the HCDR3 includes an amino acid sequence as set forth in SEQ ID NO: 9;
(3) the HCDR1 includes an amino acid sequence as set forth in SEQ ID NO: 16, the HCDR2 includes an amino acid sequence as set forth in SEQ ID NO: 15, and the HCDR3 includes an amino acid sequence as set forth in SEQ ID NO: 14;
(4) the HCDR1 includes an amino acid sequence as set forth in SEQ ID NO: 21, the HCDR2 includes an amino acid sequence as set forth in SEQ ID NO: 20, and the HCDR3 includes an amino acid sequence as set forth in SEQ ID NO: 19;
(5) the HCDR1 includes an amino acid sequence as set forth in SEQ ID NO: 26, the HCDR2 includes an amino acid sequence as set forth in SEQ ID NO: 25, and the HCDR3 includes an amino acid sequence as set forth in SEQ ID NO: 24;
(6) the HCDR1 includes an amino acid sequence as set forth in SEQ ID NO: 31, the HCDR2 includes an amino acid sequence as set forth in SEQ ID NO: 30, and the HCDR3 includes an amino acid sequence as set forth in SEQ ID NO: 29; and
(7) the HCDR1 includes an amino acid sequence as set forth in SEQ ID NO: 36, the HCDR2 includes an amino acid sequence as set forth in SEQ ID NO: 35, and the HCDR3 includes an amino acid sequence as set forth in SEQ ID NO: 34.

In some embodiments, the antigen binding protein includes an H-FR1, a C-terminal of the H-FR1 is connected to an N-terminal of the HCDR1 directly or indirectly, and the H-FR1 includes an amino acid sequence as set forth in any one of SEQ ID NO: 4, SEQ ID NO: 12, SEQ ID NO: 17, SEQ ID NO: 22, SEQ ID NO: 27, SEQ ID NO: 32, and SEQ ID NO: 37.

In some embodiments, the antigen binding protein includes an H-FR2, the H-FR2 is located between the HCDR1 and the HCDR2, and the H-FR2 includes an amino acid sequence as set forth in SEQ ID NO: 5.

In some embodiments, the antigen binding protein includes an H-FR3, the H-FR3 is located between between the HCDR2 and the HCDR3, and the H-FR3 includes an amino acid sequence as set forth in SEQ ID NO: 6.

In some embodiments, the antigen binding protein includes an H-FR4, an N-terminal of the H-FR4 is connected to a C-terminal of the HCDR3 directly or indirectly, and the H-FR4 includes an amino acid sequence as set forth in SEQ ID NO: 7.

In some embodiments, the antigen binding protein includes an antibody heavy chain variable region VH, and the VH includes an amino acid sequence as set forth in any one of SEQ ID NO: 8, SEQ ID NO: 13, SEQ ID NO: 69, and SEQ ID NO: 70.

In some embodiments, the antigen binding protein includes an antibody heavy chain variable region VH, and the VH includes an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

In some embodiments, the antigen binding protein includes an antibody or an antigen binding fragment thereof.

In some embodiments, the antigen binding fragment includes Fab, Fab', F(ab)₂, an Fv fragment, F(ab')₂, scFv, di-scFv, VHH, and/or dAb.

In some embodiments, the antibody is selected from a group consisting of a monoclonal antibody, a chimeric antibody, a humanized antibody, and a fully human antibody.

In some embodiments, the antigen binding fragment is a VHH, and the VHH includes an amino acid sequence as set forth in any one of SEQ ID NO: 8, SEQ ID NO: 13, SEQ ID NO: 69, and SEQ ID NO: 70.

In some embodiments, the antigen binding fragment is a VHH, and the VHH includes an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

In some embodiments, the antigen binding protein is capable of competing with a reference antibody for binding to MSLN (mesothelin) protein, where the reference antibody includes an antibody heavy chain variable region VH, the VH of the reference antibody includes an HCDR1, an HCDR2, and an HCDR3, and the reference antibody includes any one group of amino acid sequences selected from:
(1) the HCDR1 includes an amino acid sequence as set forth in SEQ ID NO: 3, the HCDR2 includes an amino acid sequence as set forth in SEQ ID NO: 2, and the HCDR3 includes an amino acid sequence as set forth in SEQ ID NO: 1;
(2) the HCDR1 includes an amino acid sequence as set forth in SEQ ID NO: 11, the HCDR2 includes an amino acid sequence as set forth in SEQ ID NO: 10, and the HCDR3 includes an amino acid sequence as set forth in SEQ ID NO: 9;
(3) the HCDR1 includes an amino acid sequence as set forth in SEQ ID NO: 16, the HCDR2 includes an amino acid sequence as set forth in SEQ ID NO: 15, and the HCDR3 includes an amino acid sequence as set forth in SEQ ID NO: 14;
(4) the HCDR1 includes an amino acid sequence as set forth in SEQ ID NO: 21, the HCDR2 includes an amino acid sequence as set forth in SEQ ID NO: 20, and the HCDR3 includes an amino acid sequence as set forth in SEQ ID NO: 19;
(5) the HCDR1 includes an amino acid sequence as set forth in SEQ ID NO: 26, the HCDR2 includes an amino acid sequence as set forth in SEQ ID NO: 25, and the HCDR3 includes an amino acid sequence as set forth in SEQ ID NO: 24;
(6) the HCDR1 includes an amino acid sequence as set forth in SEQ ID NO: 31, the HCDR2 includes an amino acid sequence as set forth in SEQ ID NO: 30, and the HCDR3 includes an amino acid sequence as set forth in SEQ ID NO: 29; and
(7) the HCDR1 includes an amino acid sequence as set forth in SEQ ID NO: 36, the HCDR2 includes an amino acid sequence as set forth in SEQ ID NO: 35, and the HCDR3 includes an amino acid sequence as set forth in SEQ ID NO: 34.

In some embodiments, the antigen binding protein includes an antibody heavy chain constant region, the antibody heavy chain constant region is derived from IgG.

In some embodiments, the antigen binding protein includes an antibody heavy chain constant region, the antibody heavy chain constant region is derived from human IgG.

In some embodiments, the antigen binding protein includes an antibody heavy chain constant region, the antibody heavy chain constant region is derived from human IgG1.

In some embodiments, the heavy chain constant region of the antigen binding protein includes an Fc region of IgG.

In some embodiments, the Fc region includes an amino acid sequence as set forth in SEQ ID NO: 54.

In another aspect, the present application further provides a chimeric antigen receptor which includes a targeting moiety, the targeting moiety includes the antigen binding protein.

In some embodiments, the chimeric antigen receptor includes a costimulatory domain, the costimulatory domain includes a costimulatory domain derived from one or more proteins selected from a group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

In some embodiments, the costimulatory domain of the chimeric antigen receptor is an intracellular costimulatory signaling region derived from 4-1BB.

In some embodiments, the costimulatory domain of the chimeric antigen receptor includes an amino acid sequence as set forth in SEQ ID NO: 39.

In some embodiments, the chimeric antigen receptor includes an intracellular signaling domain, and the intracellular signaling domain includes an intracellular signaling domain derived from one or more proteins selected from a group consisting of: CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP-12, and a domain containing at least one ITAM.

In some embodiments, the intracellular signaling domain of the chimeric antigen receptor is a signaling domain derived from CD3ζ.

In some embodiments, the intracellular signaling domain of the chimeric antigen receptor includes an amino acid sequence as set forth in SEQ ID NO: 41.

In some embodiments, the chimeric antigen receptor includes a transmembrane region, and the transmembrane region includes a transmembrane domain derived from one or more proteins selected from a group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

In some embodiments, the transmembrane region of the chimeric antigen receptor is a transmembrane region derived from CD8.

In some embodiments, the transmembrane region of the chimeric antigen receptor includes an amino acid sequence as set forth in SEQ ID NO: 40.

In some embodiments, the chimeric antigen receptor includes a hinge region between the targeting moiety and the transmembrane region, and the hinge region includes a hinge region derived from one or more proteins selected from a group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

In some embodiments, the hinge region of the chimeric antigen receptor is a hinge region derived from CD8.

In some embodiments, the hinge region of the chimeric antigen receptor includes an amino acid sequence as set forth in SEQ ID NO: 42.

In some embodiments, the chimeric antigen receptor further includes a signal peptide.

In some embodiments, the signal peptide of the chimeric antigen receptor is derived from a signal peptide of CD8 protein.

In some embodiments, the signal peptide of the chimeric antigen receptor includes an amino acid sequence as set forth in SEQ ID NO: 43.

In some embodiments, the chimeric antigen receptor further includes a low-density lipoprotein receptor-related protein or a fragment thereof.

In some embodiments, the low-density lipoprotein receptor-related protein or the fragment thereof includes one or more selected from a group consisting of low-density lipoprotein receptor-related proteins 1-12 and functional fragments thereof.

In some embodiments, the low-density lipoprotein receptor-related protein or the fragment thereof is low-density lipoprotein receptor-related proteins 5 and/or 6 or fragments thereof.

In some embodiments, the low-density lipoprotein receptor-related protein or the fragment thereof includes an amino acid sequence as set forth in SEQ ID NO: 44.

In some embodiments, the chimeric antigen receptor includes an amino acid sequence as set forth in any one of SEQ ID NO: 47, SEQ ID NO: 48, SEQ ID NO: 49, SEQ ID NO: 50, SEQ ID NO: 51, SEQ ID NO: 52, and SEQ ID NO: 53.

In another aspect, the present application further provides a polypeptide, which includes the antigen binding protein.

In another aspect, the present application further provides one or more isolated nucleic acid molecules, which encode the isolated antigen binding protein and/or the chimeric antigen receptor.

In some embodiments, the nucleic acid molecules include a promoter.

In some embodiments, the promoter is a constitutive promoter.

In some embodiments, the promoter is an EF1α promoter.

In another aspect, the present application further provides a vector, which includes the nucleic acid molecules.

In some embodiments, the vector includes a viral vector.

In some embodiments, the vector includes a lentiviral vector.

In another aspect, the present application further provides a cell, which includes the antigen binding protein, the chimeric antigen receptor, the nucleic acid molecules, and/or the vector.

In some embodiments, the cell is an immune effector cell.

In some embodiments, the cell includes T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

In some embodiments, the cell is a T cell.

In some embodiments, the cell includes and/or expresses a low-density lipoprotein receptor-related protein or a fragment thereof.

In some embodiments, the low-density lipoprotein receptor-related protein or the fragment thereof includes one or more selected from a group consisting of low-density lipoprotein receptor-related proteins 1-12 and functional fragments thereof.

In some embodiments, the low-density lipoprotein receptor-related protein or the fragment thereof is low-density lipoprotein receptor-related proteins 5 and/or 6 or fragments thereof.

In some embodiments, the low-density lipoprotein receptor-related protein or the fragment thereof includes an amino acid sequence as set forth in SEQ ID NO: 44.

In another aspect, the present application further provides a method for preparing a modified immune effector cell, which includes culturing the cell under a condition enabling the expression of the antigen binding protein and/or the chimeric antigen receptor.

In another aspect, the present application further provides a method for preparing a modified immune effector cell, which includes introducing the vector into the immune effector cell.

In another aspect, the present application further provides a pharmaceutical composition, which includes the isolated antigen binding protein, the chimeric antigen receptor, the polypeptide, the nucleic acid molecules, the vector, and/or the cell, as well as optionally a pharmaceutically acceptable carrier.

In another aspect, the present application further provides use of the isolated antigen binding protein, the chimeric antigen receptor, the polypeptide, the nucleic acid molecules, the vector, the cell, and/or the pharmaceutical composition in the preparation of a drug for preventing, treating and/or alleviating a disease or a disorder associated with abnormal expression of MSLN.

In some embodiments, the disease or disorder associated with abnormal expression of MSLN includes a tumor.

In some embodiments, the tumor includes a solid tumor.

In some embodiments, the tumor includes a non-solid tumor.

In some embodiments, the tumor includes an MSLN antigen-expressing tumor.

In some embodiments, the tumor includes ovarian cancer, pancreatic cancer, gastric cancer, mesothelioma, bile duct cancer, triple-negative breast cancer, and/or endometrial cancer.

In another aspect, the present application further provides a method for preventing, treating and/or alleviating a disease or a disorder associated with abnormal expression of MSLN, which includes administering to a subject in need thereof the isolated antigen binding protein, the chimeric antigen receptor, the polypeptide, the nucleic acid molecules, the vector, the cell, and/or the pharmaceutical composition.

In some embodiments, the disease or disorder associated with abnormal expression of MSLN includes a tumor.

In some embodiments, the tumor includes a solid tumor.

In some embodiments, the tumor includes a non-solid tumor.

In some embodiments, the tumor includes an MSLN antigen-expressing tumor.

In some embodiments, the tumor includes ovarian cancer, pancreatic cancer, gastric cancer, mesothelioma, bile duct cancer, triple-negative breast cancer, and/or endometrial cancer.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
FIG. 1 shows phage Pool ELISA results.
FIG. 2 shows selection of positive clones.
FIGs. 3A-3D show affinity test of the antigen binding protein of the present application to MSLN.
FIG. 4 shows the binding activity of the antigen binding protein of the present application to 293T overexpressing human MSLN verified by flow cytometry.
FIG. 5 shows the nonspecific binding of the antigen binding protein of the present application to 293T, KERA, LO2, A549, and HACAT cells verified by flow cytometry.
FIG. 6 shows the results of *in vitro* repeated stimulation proliferation assays of CAR-T cells.
FIG. 7 shows the *in vitro* killing results of CAR-T cells.
FIGs. 8-10 show the assay results of the *in vitro* cytokine release of CAR-T cells.
FIG. 11 shows the results of *in vivo* efficacy test in SK-OV3 tumor model mice.

### Detailed Description of the Embodiments

The implementation of the present application will be illustrated in the following specific examples, and other advantages and effects of the present application will be easily known by those skilled in the art from the content disclosed in the specification.

### Definition of Terms

In the present application, the term "MSLN", also known as mesothelin, or CAK1 antigen or pre-pro-megakaryocyte-potentiating factor, is a protein that presents on normal mesothelial cells and is over-expressed in some tumor cells. In the present application, the term may include MSLN protein or functionally active fragments thereof. In the present application, the term may further include homologs, analogs or variants of the MSLN protein. For example, the MSLN may include human MSLN.

In the present application, the term "isolated antigen binding protein" generally refers to a protein with antigen-binding ability that is isolated from its native state. The "isolated antigen binding protein" may include an antigen binding portion and optionally, a scaffold or framework portion that allows the antigen binding portion to adopt a conformation that facilitates the antigen-binding portion to bind to the antigen. The antigen-binding protein may include, for example, an antibody-derived protein framework region (FR) or an alternative protein framework region or an artificial framework region with a grafted variable region (CDR) or a CDR derivative. For example, the antigen binding protein may include an antibody or an antigen binding fragment thereof. For example, the antigen binding protein may bind to MSLN protein. For example, the antigen binding protein may compete with a reference antibody for binding to MSLN protein. For example, the antigen binding protein may include an antibody heavy chain variable region VH. For example, the antigen binding protein may include at least one CDR derived from an antibody heavy chain variable region VH. For example, the VH may include an HCDR3, an HCDR2, and/or an HCDR1. For example, the VH may include a framework region H-FR1, a C-terminal of the H-FR1 is connected to an N-terminal of the HCDR1 directly or indirectly. For example, the VH may include a framework region H-FR2, the H-FR2 is located between the HCDR1 and the HCDR2. For example, the VH may include a framework region H-FR3, the H-FR3 is located between the HCDR2 and the HCDR3. For example, the VH may include a framework region H-FR4, an N-terminal of the H-FR4 is connected to a C-terminal of the HCDR3. For example, the antigen binding protein may be a VHH. For example, the antigen binding protein may include an antibody heavy chain constant region, and the antibody heavy chain constant region may be derived from IgG. For example, the antibody heavy chain constant region may be derived from human IgG. For example, the antibody heavy chain constant region may be derived from human IgG1.

The term "antibody" as used includes an intact antibody and a binding fragment thereof. Generally, the fragment competes with the intact antibody from which it is derived for specifically binding to the antigen. Optionally, the antibody or the binding fragment thereof may bind to other protein chemically, or may be expressed in a form of fusion protein with other proteins. For example, the antibody may be a monoclonal antibody, a chimeric antibody, a humanized antibody, and a fully human antibody. For example, the binding protein of the antibody or the binding fragment thereof may include MSLN. For example, the antibody or the binding fragment thereof may be specific to MSLN.

The term "antigen binding fragment" refers to a part of an intact antibody and refers to the antigen determining variable region of the intact antibody. For example, the antigen binding fragment may include Fab, Fab', F(ab')2, an Fv fragment and a single-stranded Fv fragment, a tandem Fv fragment, VHH, a bispecific antibody. For example, the antigen binding fragment may be a VHH. For example, the antigen binding fragment may bind to MSLN. For example, the antigen binding fragment may be specific to MSLN.

In the present application, the term "VHH" generally refers to an antibody containing a variable antigen binding domain of a heavy chain antibody. VHH may be also referred to as nanobody (Nb) and/or single-domain antibody. For example, the VHH may bind to MSLN. For example, the VHH may be specific to MSLN.

In the present application, the antibody may include at least two heavy (H) chains and two light (L) chains which are connected to each other through disulfide bonds. Each heavy chain is composed of a heavy chain variable region (VH) and a heavy chain constant region. The term "heavy chain constant region" is composed of three domains CH1, CH2, and CH3. Each light chain is composed of a light chain variable region (VL) and a light chain constant region. The term "light chain constant region" is composed of one domain CL. VH and VL regions can be further subdivided into hypervariable regions called complementarity determining regions (CDR) interspersed with more conservative regions called framework regions (FR). Each of VH and VL includes three CDRs and four FRs, arranged in the following order from the amino terminus to the carboxyl terminus: FR1, CDR1, FR2, CDR2, FR3, CDR3 and FR4. The variable regions of heavy chain and light chain contain binding domains interacting with antigens. The constant region of the antibody can mediate the binding of immunoglobulins to host tissues or factors.

In the present application, the term "reference antibody" refers to an antibody that can competitively bind to the same epitope of MSLN with the isolated antigen binding protein. The reference antibody may include a heavy chain variable region VH. For example, the reference antibody may have 3 CDR sequences. For example, the VH of the reference antibody may include an HCDR1, an HCDR2, and an HCDR3. For example, the CDR sequence may be consistent with the CDR sequence of the isolated antigen binding protein.

In the present application, the term "IgG" refers to a polypeptide belonging to a class of antibodies essentially encoded by a recognized immunoglobulin gamma gene. In human, such a class includes IgG1, IgG2, IgG3, and IgG4. In mice, such a class includes IgG1, IgG2a, IgG2b, and IgG3.

In the present application, the term "chimeric antigen receptor" (CAR) generally refers to a recombinant polypeptide comprising at least an extracellular domain, a transmembrane region, and an intracellular domain that specifically bind to an antigen or a target. For example, a hinge region is included between the extracellular domain and the transmembrane region. For example, the chimeric antigen receptor may further include a low-density lipoprotein receptor-related protein or a fragment thereof. For example, the chimeric antigen receptor may include a signal peptide. The binding of the extracellular domain of CAR to the target antigen on the surface of target cells leads to CAR clustering and delivery of activation stimulus to the CAR-containing cells. CAR redirects the specificity of immune effector cells and triggers proliferation, cytokine production, phagocytosis and/or production of molecules capable of mediating the death of cells expressing target antigens in a manner that is not dependent on major histocompatibility (MHC). For example, the extracellular domain may include the antigen binding protein above. For example, the extracellular domain may specifically bind to MSLN.

In the present application, the term "intracellular domain" refers to an intracellular domain comprising any truncated portions that are sufficient to transduce the activation signals. The intracellular domain may include an intracellular signaling region and/or a costimulatory signaling region. The term "intracellular signaling region" refers to an intracellular region that can produce a signal that promotes the immune effector function of CAR-containing cells (e.g., CART cells or CAR-expressing NK cells). For example, the intracellular signaling region may include an intracellular signaling region of one or more proteins selected from a group consisting of: CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj 14 Nef, Kaposi's sarcoma-associated herpesvirus (HSKV), DAP10, DAP-12, and a domain containing at least one ITAM. For example, the intracellular signaling region may be a signaling domain derived from CD3ζ. The term "costimulatory signaling region" refers to a portion of CAR in the intracellular signaling domain that can transduce effect signals. For example, the costimulatory signaling region may include an intracellular costimulatory signaling region derived from one or more proteins selected from a group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88. For example, the costimulatory signaling region may be an intracellular costimulatory signaling region derived from 4-1BB.

In the present application, the term "transmembrane region" refers to a domain of a peptide, a polypeptide or a protein capable of spanning the cytoplasmic membrane. These domains can be used to anchor an extracellular domain on the cell membrane. For example, the transmembrane region may include a transmembrane domain of one or more proteins selected from a group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM. For example, the transmembrane region may be derived from a transmembrane region of CD8.

In the present application, the term "hinge region" represents a part connecting the CH1 domain to the CH2 domain in an antibody heavy chain polypeptide, e.g., positions from about 216 to about 230 according to the EU numbering system to Kabat. A hinge region is generally a dimer molecule composed of two polypeptides with the same amino acid sequence. A hinge region typically includes about 25 amino acid residues that are flexible, allowing the independent motion of the antigen binding region. A hinge region can be subdivided into 3 domains: upper, middle, lower hinge domains. For example, the hinge region may include a hinge region derived from one or more proteins selected from a group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT. For example, the hinge region may be derived from a hinge region of CD8.

In the present application, the term "low-density lipoprotein receptor-related protein" refers to a cell surface protein that is an endocytic receptor. It is widely distributed in living organisms with great differences among tissues. Its main function is to take up cholesterol into cells for cell proliferation and synthesis of steroid hormones and bile salts. For example, the low-density lipoprotein receptor-related protein may be derived from any vertebrates. For example, the low-density lipoprotein receptor-related protein or the fragment thereof may be located at the C-terminal of the intracellular signaling region. For example, the low-density lipoprotein receptor-related protein or the fragment thereof may include one or more selected from a group consisting of low-density lipoprotein receptor-related proteins 1-12 and functional fragments thereof. For example, the low-density lipoprotein receptor-related protein or the fragment thereof may be low-density lipoprotein receptor-related protein 6 or a fragment thereof.

In the present application, the term "signal peptide" refers to a leader sequence at the amino terminus (N-terminus) of a nascent CAR protein which directs the nascent protein to the endoplasmic reticulum during or after translation, followed by surface expression. For example, the signal peptide is derived from a signal peptide of CD8 protein.

In the present application, the terms "polypeptide", "peptide", and "protein" are used interchangably herein and refer to a polymer of amino acid residues. This term may be used to refer to amino acid polymers in which one or more amino acid residues are synthetic chemical analogs of their corresponding naturally occurring amino acids, as well as to naturally occurring amino acid polymers, those containing modified residues, and non-naturally occurring amino acid polymers. For example, the polypeptide may include the antigen binding protein.

In the present application, the term "nucleic acid molecule" includes DNA molecules and RNA molecules. A nucleic acid molecule may be single or double stranded, preferably double-stranded DNA. The term "promoter" generally refers to a DNA sequence that can regulate the expression of a selected DNA sequence operably linked to the promoter, thereby affecting the expression of the selected DNA sequence in the cell. For example, the nucleic acid molecule may encode the antigen binding protein and/or the chimeric antigen receptor. For example, the nucleic acid molecule may include a promoter. For example, the promoter may be a constitutive promoter. For example, the promoter may be an EF1α promoter.

In the present application, the term "vector" generally refers to a molecule on which one or more nucleic acid molecules of the present application can be attached. For example, the vector may be a viral vector. For example, the vector may be a lentiviral vector.

In the present application, the term "cell" refers to a cell to which nucleic acid can be transfected. The term "cell" includes prokaryotic cells for plasmid propagation, and eukaryotic cells for nucleic acid expression and encoded polypeptide production. For example, the cell may include the antigen binding protein, the nucleic acid molecule, and/or the vector. For example, the cell may be an immune effector cell. The term "immune effector cell" generally refers to immune cells that participate in immune responses and perform effector functions. For example, the exercise of effector functions may include removal of foreign antigens or promotion of immune effector responses, etc. For example, the immune effector cell may include T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells. For example, the immune effector cell may be a T cell.

In the present application, the term "pharmaceutical composition" generally refers to chemical or biological compositions suitable for administration to mammalian subjects. For example, the pharmaceutical composition may include the antigen binding protein, the chimeric antigen receptor, the polypeptide, the nucleic acid molecule, the vector and/or the cell, and optionally a pharmaceutically acceptable carrier. The pharmaceutical composition may be used for preventing, treating and/or alleviating a disease or a disorder associated with abnormal expression of MSLN. For example, the disease or disorder associated with abnormal expression of MSLN may include a tumor. For example, the tumor includes a solid tumor and/or a non-solid tumor.

In the present application, the term "specifically binding to" or "specific" generally refers to measurable and reproducible interactions, such as the binding between targets and antibodies, which can determine the presence of the targets in the presence of a heterogeneous population of molecules, including biomolecules. For example, an antibody specifically binding to a target (which may be an epitope) is an antibody that binds to the target with greater affinity, avidity, easier, and/or for a greater duration than it binds to other targets. In some embodiments, the antibody specifically binds to the epitope on the protein, and the epitope is conservative among proteins of different species. In some embodiments, specifical binding may include, but does not require exclusive binding.

In the present application, the term "subject" generally refers to human or non-human animals, including but not limited to cat, dog, horse, pig, cow, sheep, rabbit, mouse, rat, or monkey.

The protein, polypeptide and/or amino acid sequences involved in the present application should also be understood to include at least the following ranges: variants or homologs having the same or similar functions as those of the protein or polypeptide.

In the present application, the variants may be, e.g., proteins or polypeptides with one or more amino acid substitutions, deletions or additions in the amino acid sequence of the protein and/or the polypeptide (e.g., specifically binding to MSLN). For example, the functional variants may include proteins or polypeptides with amino acid changes by at least 1, for example, 1-30, 1-20 or 1-10, and further for example, 1, 2, 3, 4 or 5 amino acid substitutions, deletions and/or insertions. The functional variants can substantially maintain the biological properties of the protein or the polypeptide before change (e.g., substitution, deletion or addition). For example, the functional variants can maintain at least 60%, 70%, 80%, 90%, or 100% of the biological activity (e.g., antigen binding ability) of the protein or the polypeptide before change. For example, the substitution may be conservative substitution.

In the present application, the homolog may be a protein or polypeptide having at least about 85% (e.g., at least about 85%, about 90%, about 91%, about 92%, about 93%, about 94%, about 95%, about 96%, about 97%, about 98%, about 99% or higher) sequence homology with the amino acid sequence of the protein and/or the polypeptide (for example, specifically binding to MSLN).

In the present application, the homology generally refers to the similarity, likeness or correlation between two or more sequences. The "percentage of sequence homology" can be calculated by: comparing two sequences to be aligned in a comparison window to determine the number of positions where the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present in both sequences so as to obtain the number of matching positions, dividing the number of matching positions by the total number of positions in the comparison window (i.e., window size), and multiplying the result by 100, to generate the percentage of sequence homology. The alignment for determining the percentage of sequence homology can be achieved in a variety of ways known in the art, for example, by using publicly available computer softwares, such as BLAST, BLAST-2, ALIGN or Megalign (DNASTAR) softwares. A person skilled in the art can determine appropriate parameters for aligning sequences, including any algorithms needed to achieve the maximum alignment over the full-length sequence range being compared or within the target sequence region. The homology can also be determined by the following methods: FASTA and BLAST. A description of FASTA algorithm can be found in "Improved tools for biological sequence comparison" to W. R. Pearson and D. J. Lipman, Proc. Natl. Acad. Sci., 85: 2444-2448, 1988; and "Rapid and Sensitive Protein Similarity Searches" to D. J. Lipman and W. R. Pearson, Science, 227: 1435-1441, 1989. A description of BLAST algorithm can be found in "Basic Local Alignment Search Tool" to S. Altschul, W. Gish, W. Miller, E. W. Myers and D. Lipman, Journal of Molecular Biology, 215: 403-410, 1990.

In the present application, the term "include" generally refers to the meaning of include, encompass, contain or embrace. In some cases, it also indicates the meaning of "is", or "composed of ......".

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below a specified value.

### Detailed Description of the Invention

### Isolated antigen binding protein

In the present application, the antigen binding protein may include an antibody or an antigen binding fragment thereof. In the present application, the antigen binding fragment may include Fab, Fab', F(ab)₂, an Fv fragment, F(ab')₂, scFv, di-scFv, VHH, and/or dAb. In the present application, the antibody may include a monoclonal antibody, a chimeric antibody, a humanized antibody, and a fully human antibody.

### CDR

A CDR of an antibody, also known as a complementary determining region, is a part of a variable region. Amino acid residues in this region can be in contact with antigens or antigen epitopes. The CDR of an antibody can be determined through a variety of coding systems, such as CCG, Kabat, Chothia, IMGT, AbM, or a combination of Kabat/Chothia, etc. These numbering systems are known in the art, particularly see, e.g., http://www.bioinf.org.uk/abs/index.html#kabatnum. Those skilled in the art can determine the CDR regions using different numbering systems based on the sequence and structure of the antibody. There may be differences among the CDR regions when using different numbering systems. In the present application, the CDR encompasses CDR sequences divided according to any CDR division method; it also encompasses variants thereof, wherein the variants include substitution, deletion and/or addition of one or more amino acids in the amino acid sequence of the CDR, for example, substitution, deletion and/or insertion of 1-30, 1-20 or 1-10, further for example, 1, 2, 3, 4, 5, 6, 7, 8 or 9 amino acids; and it also encompasses homologs thereof, wherein the homologs may be amino acid sequences having at least about 85% (e.g., at least about 85%, about 90%, about 91%, about 92%, about 93%, about 94%, about 95%, about 96%, about 97%, about 98%, about 99% or higher) sequence homology with the amino acid sequence of the CDR. In the present application, the isolated antigen binding protein is defined by Kabat numbering system.

In the present application, the isolated antigen binding protein may include at least one CDR in an antibody heavy chain variable region VH. For example, the VH may include an amino acid sequence as set forth in any one of SEQ ID NO: 8, SEQ ID NO: 13, SEQ ID NO: 69, and SEQ ID NO: 70.

In the present application, the antigen binding protein may include an HCDR3, and the HCDR3 includes an amino acid sequence as set forth in any one of SEQ ID NO: 1, SEQ ID NO: 9, SEQ ID NO: 65, and SEQ ID NO: 66.

For example, the HCDR3 may include an amino acid sequence as set forth in any one of SEQ ID NO: 1, SEQ ID NO: 9, SEQ ID NO: 14, SEQ ID NO: 19, SEQ ID NO: 24, SEQ ID NO: 29, and SEQ ID NO: 34.

In the present application, the isolated antigen binding protein may include an HCDR2, and the HCDR2 may include an amino acid sequence as set forth in SEQ ID NO: 67. For example, the HCDR2 may include an amino acid sequence as set forth in any one of SEQ ID NO: 2, SEQ ID NO: 10, SEQ ID NO: 15, SEQ ID NO: 20, SEQ ID NO: 25, SEQ ID NO: 30, and SEQ ID NO: 35.

In the present application, the isolated antigen binding protein may include an HCDR1, and the HCDR1 may include an amino acid sequence as set forth in SEQ ID NO: 68 (X₁X₂X₃MG, wherein, X₁ is N, R, S, T or Y, X₂ is N or Y, and X₃ is A, N or V). For example, the HCDR1 may include an amino acid sequence as set forth in any one of SEQ ID NO: 3, SEQ ID NO: 11, SEQ ID NO: 16, SEQ ID NO: 21, SEQ ID NO: 26, SEQ ID NO: 31, and SEQ ID NO: 36.

In the present application, the isolated antigen binding protein may include an HCDR1, an HCDR2, and an HCDR3, and the HCDR1 may include an amino acid sequence as set forth in SEQ ID NO: 68 (X₁X₂X₃MG, wherein, X₁ is N, R, S, T or Y, X₂ is N or Y, and X₃ is A, N or V), the HCDR2 may include an amino acid sequence as set forth in SEQ ID NO: 67, and the HCDR3 may include an amino acid sequence as set forth in any one of SEQ ID NO: 1, SEQ ID NO: 9, SEQ ID NO: 65, and SEQ ID NO: 66.

In the present application, the isolated antigen binding protein may include an HCDR1, an HCDR2, and an HCDR3, and the HCDR1, HCDR2, and HCDR3 may include any one group of amino acid sequences selected from:
(1) the HCDR1 includes an amino acid sequence as set forth in SEQ ID NO: 3, the HCDR2 includes an amino acid sequence as set forth in SEQ ID NO: 2, and the HCDR3 includes an amino acid sequence as set forth in SEQ ID NO: 1;
(2) the HCDR1 includes an amino acid sequence as set forth in SEQ ID NO: 11, the HCDR2 includes an amino acid sequence as set forth in SEQ ID NO: 10, and the HCDR3 includes an amino acid sequence as set forth in SEQ ID NO: 9;
(3) the HCDR1 includes an amino acid sequence as set forth in SEQ ID NO: 16, the HCDR2 includes an amino acid sequence as set forth in SEQ ID NO: 15, and the HCDR3 includes an amino acid sequence as set forth in SEQ ID NO: 14;
(4) the HCDR1 includes an amino acid sequence as set forth in SEQ ID NO: 21, the HCDR2 includes an amino acid sequence as set forth in SEQ ID NO: 20, and the HCDR3 includes an amino acid sequence as set forth in SEQ ID NO: 19;
(5) the HCDR1 includes an amino acid sequence as set forth in SEQ ID NO: 26, the HCDR2 includes an amino acid sequence as set forth in SEQ ID NO: 25, and the HCDR3 includes an amino acid sequence as set forth in SEQ ID NO: 24;
(6) the HCDR1 includes an amino acid sequence as set forth in SEQ ID NO: 31, the HCDR2 includes an amino acid sequence as set forth in SEQ ID NO: 30, and the HCDR3 includes an amino acid sequence as set forth in SEQ ID NO: 29; and
(7) the HCDR1 includes an amino acid sequence as set forth in SEQ ID NO: 36, the HCDR2 includes an amino acid sequence as set forth in SEQ ID NO: 35, and the HCDR3 includes an amino acid sequence as set forth in SEQ ID NO: 34.

### FR

In the present application, an antibody framework region FR refers to the part of an antibody variable region that exists between the more divergent (i.e., hypervariable) CDRs. Such framework regions are typically referred to as frameworks 1 to 4 (FR1, FR2, FR3, and FR4) and provide a scaffold for presenting six CDRs (three from heavy chains and three from light chains) in three-dimensional space to form an antigen-binding surface.

In the present application, the isolated antigen binding protein may include an H-FR1, and the H-FR1 may include an amino acid sequence as set forth in any one of SEQ ID NO: 4, SEQ ID NO: 12, SEQ ID NO: 17, SEQ ID NO: 22, SEQ ID NO: 27, SEQ ID NO: 32, and SEQ ID NO: 37.

In the present application, the isolated antigen binding protein may include an H-FR2, and the H-FR2 may include an amino acid sequence as set forth in SEQ ID NO: 5.

In the present application, the isolated antigen binding protein may include an H-FR3, and the H-FR3 may include an amino acid sequence as set forth in SEQ ID NO: 6.

In the present application, the isolated antigen binding protein may include an H-FR4, and the H-FR4 may include an amino acid sequence as set forth in SEQ ID NO: 7.

In the present application, the isolated antigen binding protein may include an H-FR1, an H-FR2, an H-FR3, and an H-FR4, and the H-FR1 may include an amino acid sequence as set forth in any one of SEQ ID NO: 4, SEQ ID NO: 12, SEQ ID NO: 17, SEQ ID NO: 22, SEQ ID NO: 27, SEQ ID NO: 32, and SEQ ID NO: 37, the H-FR2 may include an amino acid sequence as set forth in SEQ ID NO: 5, the H-FR3 may include an amino acid sequence as set forth in SEQ ID NO: 6, and the H-FR4 may include an amino acid sequence as set forth in SEQ ID NO: 7.

### VH/VHH

In the present application, the isolated antigen binding protein may include an antibody heavy chain variable region VH, and the VH may include an amino acid sequence as set forth in any one of SEQ ID NO: 8, SEQ ID NO: 13, SEQ ID NO: 69, and SEQ ID NO: 70. For example, the VH may include an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

In the present application, the antigen binding fragment may be a VHH, and the VHH may include an amino acid sequence as set forth in any one of SEQ ID NO: 8, SEQ ID NO: 13, SEQ ID NO: 69, and SEQ ID NO: 70. For example, the VHH may include an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

### Heavy chain constant region

In the present application, the isolated antigen binding protein may include a heavy chain constant region. The heavy chain constant region refers to a region containing at least three heavy chain constant domains CH1, CH2, and CH3. Non-restrictive exemplary heavy chain constant regions include γ, δ, and α. Non-restrictive exemplary heavy chain constant regions further include ε and µ. Each heavy chain constant region corresponds to one antibody isotype. For example, an antibody containing a γ constant region is an IgG antibody, an antibody containing a δ constant region is an IgD antibody, and an antibody containing an α constant region is an IgA antibody. In addition, an antibody containing a µ constant region is an IgM antibody, and an antibody containing an ε constant region is an IgE antibody. Some isotypes can be further subdivided into subclasses. For example, IgG antibodies include, but not limited to, IgG1 (containing a γ₁ constant region), IgG2 (containing a γ₂ constant region), IgG3 (containing a γ₃ constant region), and IgG4 (containing a γ₄ constant region) antibodies; IgA antibodies include, but not limited to, IgA1 (containing an α₁ constant region) and IgA2 (containing an α₂ constant region) antibodies; and IgM includes, but not limited to, IgM1 and IgM2.

In the present application, the isolated antigen binding protein may include an antibody heavy chain constant region, and the antibody heavy chain constant region may be derived from IgG. In the present application, the isolated antigen binding protein may include an antibody heavy chain constant region, and the antibody heavy chain constant region may be derived from human IgG. In the present application, the isolated antigen binding protein may include an antibody heavy chain constant region, and the antibody heavy chain constant region may be derived from human IgG1. In the present application, the heavy chain constant region of the antigen binding protein may include an Fc region of IgG. For example, the Fc region may include an amino acid sequence as set forth in SEQ ID NO: 54.

### Chimeric antigen receptor

In another aspect, the present application further provides a chimeric antigen receptor (CAR), and the chimeric antigen receptor (CAR) may include a targeting moiety binding to MSLN protein. For example, the targeting moiety binding to MSLN protein may be the antigen binding protein of the present application.

For example, the CAR of the present application may include a VHH, and the VHH may include an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

In the present application, the CAR may also include an intracellular domain in addition to an extracellular targeting moiety that binds to the MSLN protein.

In the present application, the CAR may include an intracellular costimulatory signaling region which can provide stimulatory signals. For example, the costimulatory signaling region may include an intracellular costimulatory signaling region of one or more proteins selected from a group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

For example, the costimulatory signaling region may be an intracellular costimulatory signaling region derived from 4-1BB. For example, the costimulatory signaling region may include an amino acid sequence as set forth in SEQ ID NO: 39.

In some cases, the CAR may include an intracellular signaling region which may include a domain containing at least one ITAM motif. The intracellular signaling domain can transmit activation signals into the cells. For example, the intracellular signaling region may include an intracellular signaling region derived from one or more proteins selected from a group consisting of CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP-12, and other domains containing at least one ITAM.

For example, the intracellular signaling region may be a signaling domain derived from CD3ζ. For example, the intracellular signaling region may include an amino acid sequence as set forth in SEQ ID NO: 41.

In some cases, the CAR may include a transmembrane domain, and the transmembrane domain is a sequence in the cell surface protein that spans the cell membrane and may include a hydrophobic alpha helix. The transmembrane domain may be derived from any type I transmembrane protein. The transmembrane domain may be predicted as a synthetic sequence for forming the hydrophobic helix. For example, the transmembrane region may include a transmembrane domain derived from one or more proteins selected from a group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

For example, the transmembrane region may be a transmembrane region derived from CD8. For example, the transmembrane region may include an amino acid sequence as set forth in SEQ ID NO: 40.

In some cases, the CAR may include a hinge region, the hinge region may be located between the extracellular targeting moiety and the transmembrane domain. For example, the hinge region may include a hinge region of one or more proteins selected from a group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

For example, the hinge region may be a hinge region derived from CD8. For example, the hinge region may include an amino acid sequence as set forth in SEQ ID NO: 42.

In the present application, the CAR may further include a signal peptide at the N-terminal of the targeting moiety that binds to the MSLN protein. For example, the signal peptide may be a signal peptide derived from CD8 protein. For example, the signal peptide may include an amino acid sequence as set forth in SEQ ID NO: 43.

In the present application, the CAR may further include a low-density lipoprotein receptor-related protein or a fragment thereof. For example, the low-density lipoprotein receptor-related protein or the fragment thereof may be located at the C-terminal of the CAR. For example, the low-density lipoprotein receptor-related protein or the fragment thereof may include low-density lipoprotein receptor-related proteins 1-12 and functional fragments thereof. For example, the low-density lipoprotein receptor-related protein or the fragment thereof may be low-density lipoprotein receptor-related proteins 5 and/or 6 or fragments thereof. For example, the low-density lipoprotein receptor-related protein or the fragment thereof may include an amino acid sequence as set forth in SEQ ID NO: 44. For example, the nucleic acid molecule which encodes the low-density lipoprotein receptor-related protein or the fragment thereof may include a nucleotide sequence as set forth in SEQ ID NO: 45.

In the present application, the sequence of the low-density lipoprotein receptor-related protein or the fragment thereof in the CAR may be connected to the the C-terminal sequence of the CAR via a self-cleaving peptide (e.g., 2A peptides such as T2A, P2A, and E2A). For example, the low-density lipoprotein receptor-related protein or the fragment thereof may be connected to the C terminal of the intracellular signaling region via T2A. For example, the cleaving peptide may include an amino acid sequence as set forth in SEQ ID NO: 46.

In the present application, the CAR may include, sequentially from N-terminal to C-terminal, a targeting moiety binding to MSLN protein (for example, the antigen binding protein, further for example, the VHH of the present application), a hinge region, a transmembrane domain, a costimulatory signaling region, and an intracellular signaling region. For example, the CAR may include, sequentially from N-terminal to C-terminal, a VHH, a hinge region derived from CD8, a transmembrane region derived from CD8, a costimulatory signaling region derived from 4-1BB, and an intracellular signaling region derived from CD3ζ, and the VHH may include an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

In the present application, the CAR may include, sequentially from N-terminal to C-terminal, a targeting moiety binding to MSLN protein (for example, the antigen binding protein, further for example, the VHH of the present application), a hinge region, a transmembrane domain, a costimulatory signaling region, an intracellular signaling region, and a low-density lipoprotein receptor-related protein or a fragment thereof. For example, the CAR may include, sequentially from N-terminal to C-terminal, a VHH, a hinge region derived from CD8, a transmembrane region derived from CD8, a costimulatory signaling region derived from 4-1BB, an intracellular signaling region derived from CD3ζ, and a low-density lipoprotein receptor-related protein or a fragment thereof containing an amino acid sequence as set forth in SEQ ID NO: 44, and the VHH may include an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

In the present application, the CAR may include, sequentially from N-terminal to C-terminal, a signal peptide, a targeting moiety binding to MSLN protein (for example, the antigen binding protein, further for example, the VHH of the present application), a hinge region, a transmembrane domain, a costimulatory signaling region, and an intracellular signaling region.

In the present application, the CAR may include, sequentially from N-terminal to C-terminal, a signal peptide, a targeting moiety binding to MSLN protein (for example, the antigen binding protein, further for example, the VHH of the present application), a hinge region, a transmembrane domain, a costimulatory signaling region, an intracellular signaling region, and a low-density lipoprotein receptor-related protein or a fragment thereof.

For example, the chimeric antigen receptor may include an amino acid sequence as set forth in SEQ ID NO: 47. For example, the chimeric antigen receptor may include an amino acid sequence as set forth in SEQ ID NO: 48. For example, the chimeric antigen receptor may include an amino acid sequence as set forth in SEQ ID NO: 49. For example, the chimeric antigen receptor may include an amino acid sequence as set forth in SEQ ID NO: 50. For example, the chimeric antigen receptor may include an amino acid sequence as set forth in SEQ ID NO: 51. For example, the chimeric antigen receptor may include an amino acid sequence as set forth in SEQ ID NO: 52. For example, the chimeric antigen receptor may include an amino acid sequence as set forth in SEQ ID NO: 53.

### Nucleic acid molecules

In another aspect, the present application further provides one or more nucleic acid molecules, which may be isolated nucleotides, deoxynucleotides, and/ ribonucleotides of any length, and may encode the isolated antigen binding protein and/or the chimeric antigen receptor.

For example, the nucleic acid molecules may include a promoter. For example, the promoter may be a constitutive promoter. For example, the promoter may be an EF1α promoter.

In another aspect, the present application further provides one or more nucleic acid molecules, which include sequences capable of expressing the chimeric antigen receptor and the low-density lipoprotein receptor-related protein or the fragment thereof in the cell. In the present application, the nucleic acid sequence which encodes the chimeric antigen protein may be linked to the nucleic acid sequence which encodes the low-density lipoprotein receptor-related protein or the fragment thereof via a cleaving peptide.

### Vector

In another aspect, the present application further provides a vector, which may include the nucleic acid molecules. The vector can make the genetic elements it carries be expressed in a host cell by transforming, transducing or transfecting the host cell. For example, the vector may include promoters, transcriptons, enhancers, replicons, selective elements, and reporter genes. For example, the vector may include ingredients that help its entry into cells. In order to enable the nucleic acid molecules to replicate in the vector, the nucleic acid molecules may also include long terminal repeats at the 5' end and the 3' end.

For example, the vector may be a viral vector. For example, the vector may be a lentiviral vector.

### Cell

In another aspect, the present application further provides a cell, which may include the isolated antigen binding protein, the chimeric antigen receptor, the nucleic acid molecules, and/or the vector. The cell may include the offsprings of a single cell. Due to natural, accidental or intentional mutations, the offsprings may not necessarily be exactly the same as the original parent cells (in the form of the total DNA complement or in the genome).

In some embodiments, the cell may be an immune effector cell. In some embodiments, the cell may include T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

In some embodiments, the cell may be a T cell.

In the present application, the cell may include and/or express the CAR. In the present application, the cell may include and/or express the CAR, and the low-density lipoprotein receptor-related protein or the fragment thereof.

### Pharmaceutical composition

In another aspect, the present application further provides a pharmaceutical composition, which may include the isolated antigen binding protein, the chimeric antigen receptor, the nucleic acid molecules, the vector, and/or the cell, as well as optionally a pharmaceutically acceptable adjuvant.

In some embodiments, the pharmaceutical composition may further include one or more (pharmaceutically effective) carriers, stabilizers, excipients, diluents, solubilizers, surfactants, emulsifiers and/or preservatives and other suitable preparations. The acceptable ingredients of the composition are non-toxic to the recipient at the dosage and concentration used. The pharmaceutical composition of the present application may include liquid, frozen and lyophilized compositions.

In some embodiments, the pharmaceutically acceptable adjuvant may include any and all of the solvents, dispersion media, coatings, isotonic agents and absorption delaying agents that are compatible with the medication, which are generally safe, non-toxic and neither biologically nor otherwise undesirable.

In some embodiments, the pharmaceutical composition may be administered parenterally, transdermally, intraperitoneally, intra-arterially, intrathecally and/or intranasally or directly injected into tissues. For example, the pharmaceutical composition may be administered to patients or subjects by means of infusion or injection. In some embodiments, the pharmaceutical composition may be administered in different ways, such as intravenously, intraperitoneally, subcutaneously, intramuscularly, topically or intradermally.

### Preparation method

In another aspect, the present application further provides a method for preparing the isolated antigen binding protein and/or the chimeric antigen receptor. The method may include culturing the cell under a condition enabling the expression of the antigen receptor and/or the chimeric antigen receptor.

The present application further provides a method for preparing a modified immune effector cell, which may include introducing the vector into immune cells.

### Use

In another aspect, the present application further provides use of the isolated antigen binding protein, the chimeric antigen receptor, the nucleic acid molecule, the vector, the cell, and/or the pharmaceutical composition in the preparation of a drug for preventing, alleviating and/or treating a disease and/or a disorder.

In another aspect, the present application further provides a method for preventing, alleviating, and/or treating a disease and/or a disorder, the method may include administering to a subject the isolated antigen binding protein, the chimeric antigen receptor, the nucleic acid molecule, the vector, the cell and/or the pharmaceutical composition.

In another aspect, the present application further provides the isolated antigen binding protein, the chimeric antigen receptor, the nucleic acid molecule, the vector, the cell, and/or the pharmaceutical composition, which are used for preventing, alleviating and/or treating a disease and/or a disorder.

In the present application, the disease and/or disorder may include a disease and/or a disorder associated with abnormal expression of MSLN.

In the present application, the disease and/or disorder may include a tumor.

In the present application, the tumor may include a solid tumor and/or a non-solid tumor.

In the present application, the tumor may include blood tumor and/or lymphoma.

In the present application, the tumor may include an MSLN antigen-expressing tumor.

In the present application, the tumor may include ovarian cancer, pancreatic cancer, gastric cancer, mesothelioma, bile duct cancer, triple-negative breast cancer and/or endometrial cancer.

In the present application, the subject may include human or non-human animals.

In another aspect, the present application provides a polypeptide, which includes the isolated antigen binding protein.

In another aspect, the present application provides a kit or a drug delivery device, which includes the isolated antigen binding protein, the chimeric antigen receptor, the nucleic acid molecule, the vector, the cell and/or the pharmaceutical composition.

Without intending to be limited by any theory, the following examples are only to illustrate various technical schemes of the present application, and not intended to limit the scope of the present application.

### EXAMPLES

### Example 1 Screening of Nanobodies Targeting MSLN-His

### 1.1 Panning of those targeting MSLN-His

A full-length MSLN and its 3 domains, i.e., MSLN-D1 (AA 296-390), MSLN-D2 (AA 391-486) and MSLN-D3 (487-598) were constructed and expressed. A plate was coated with IgG-Fc and MSLN at 10 µg/mL, and placed at 4°C overnight. On the next day, the plate was washed three times with 1×PBST (PBS containing 0.05% Tween 20), blocked with 0.5% BSA for 2 h at room temperature, and washed three times with 1×PBST. 100 µl of phage library (in-house synthetic nanobody library NanoOri_1.0) was added into the IgG1-Fc wells for negative screening. After 1 h, the phages in the IgG1-Fc wells were transferred to the MSLN-His wells for positive screening. After 1.5 h, the plate was washed 10 times with 1×PBST to wash away the phages which were not bound to the antigen. Finally, the phages were eluted with Glycine-HCl at pH=2.2, with 100 µl/well, and then neutralized with Tris-HCl at pH=8.0. Half of the eluted phages were infected with TG1 in the logarithmic growth phase, and superinfected with M13KO7 after half an hour, then cultured overnight. On the next day, the phages were precipitated for the next round of screening. Similar screening process was repeated for 4 rounds. The phages were washed with 1×PBST 20 times after the positive screening in the second round, 30 times in the third round, and 40 times in the fourth round.

### 1.2 Pool ELISA

One day in advance, a 96-well plate was coated with 2 µg/mL of MSLN-His, IgG1-Fc and 0.5% BSA, which was allowed to stand overnight. On the next day, the plate was washed three times with 1×PBST, and then blocked with 0.5% BSA for 2 hrs. At the end of blocking, the plate was washed three times with 1 ×PBST. 30 µl of each round of phage library was added and incubated with shaking at room temperature for 1 h. The plate was washed three times with 1×PBST. Then, secondary antibodies, Anti-M13-HRP (Sino Biological, Item No.: 11973-MM05T-H) and Anti-Flag-HRP (abcam, Item No. ab1162), were added, incubated at room temperature for 30-60 minutes, and washed 7 times with 1×PBST. A TMB developer was added, and 2M phosphoric acid was added 3-5 min later to stop the reaction. The absorbance was read at wavelength of 450 nm.

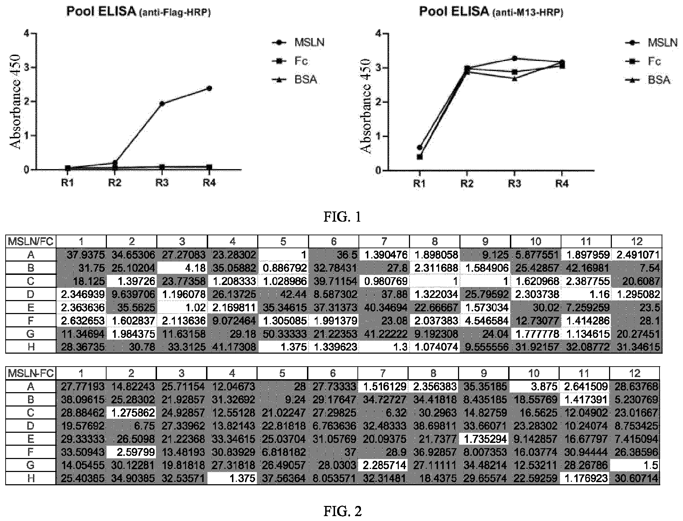

The results are shown in FIG. 1. The results show that the specific antibodies targeting MSLN-His begin to be enriched since the second round, but the curve of the anti-Flag-HRP secondary antibody shows that the enrichment is more obvious in the third and fourth rounds. Therefore, 96 monoclonal clones were picked up from each of the third and fourth rounds of bacterial culture.

### 1.3 Identification of Specific Positive Monoclones by Phage Enzyme-Linked Immunosorbent Assay (ELISA)

After 4 rounds of panning, a 2YT/carb was plated with the output reserved in the fourth round. On the next day, 192 single colonies were randomly picked up and placed in 800 µl of 2YT/carb/M13KO7, which were cultured with shaking overnight to produce phages. One day in advance, a 384-well plate was coated with 2 µg/mL of MSLN-His and IgG1-Fc which was allowed to stand overnight. On the next day, the plate was washed three times with 1×PBST, then blocked with 0.5% BSA for 2 h, and centrifuged to collect the phage supernatant. At the end of blocking, the plate was washed three times with 1 ×PBST. 30 µl of phage supernatant was added and incubated with shaking at room temperature for 1 h. The plate was washed three times with 1×PBST. Then, the secondary antibody, Anti-M13-HRP was added, incubated at room temperature for 30-60 minutes, and washed 7 times with 1×PBST. A TMB developer was added, and 2M phosphoric acid was added 3-5 min later to stop the reaction. The absorbance was read at wavelength of 450 nm. When the OD value of the sample well was 2 times or more greater than that of the control well (0.5% BSA or IgG1-Fc), it is determined to be positive. Finally, the positive phages were re-infected with TG1, and sequenced. Various clones were analyzed by the sequence comparison software BioEdit for their amino acid sequences. The clones with the same CDR1, CDR2, and CDR3 sequences were regarded as the same antibody strains.

The results are shown in FIG. 2. 192 monoclones were picked up totally, wherein the wells with MSLN/Fc>5 were considered to be positive. Then, all of the more than 130 positive clones were sequenced.

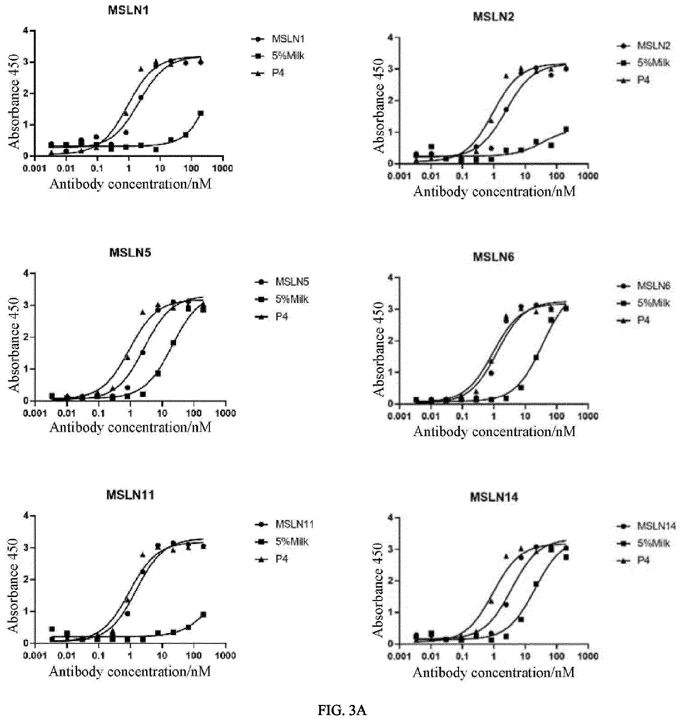

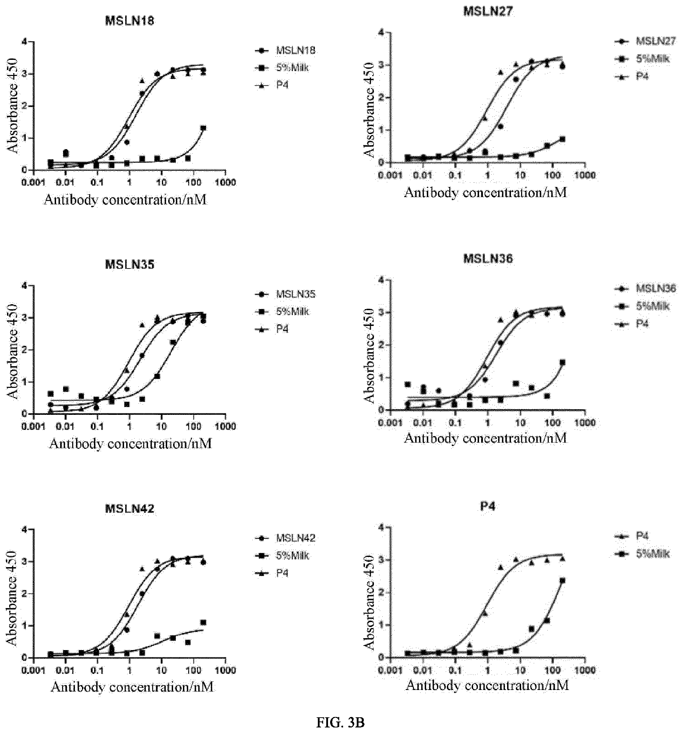

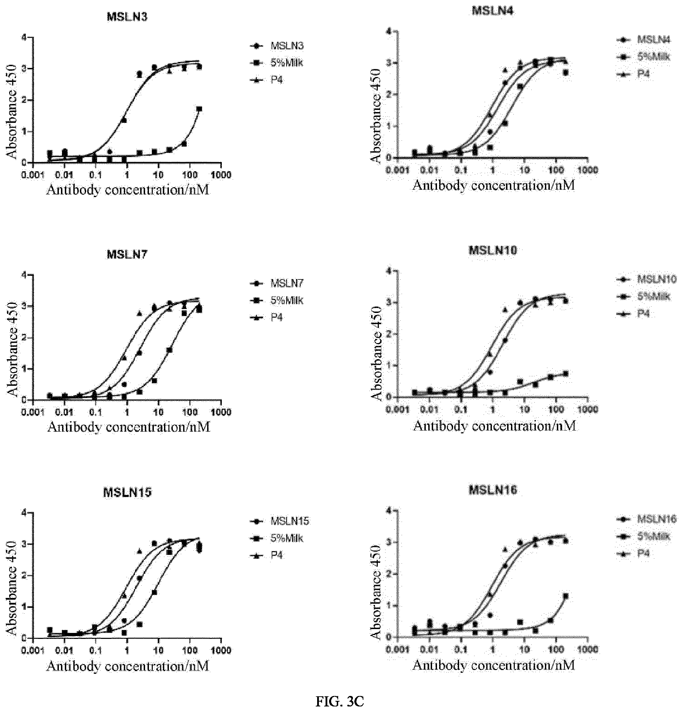

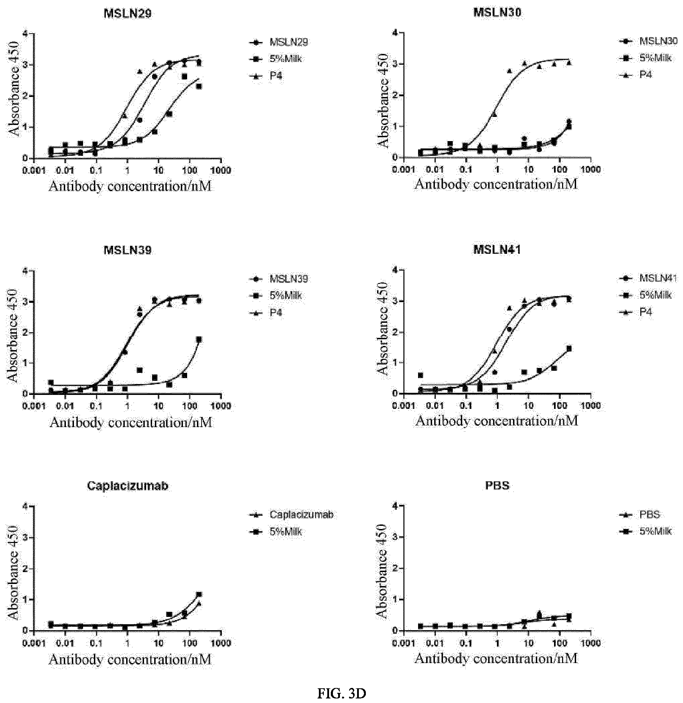

The sequencing results are shown in FIGs. 3A-3D. The sequencing results show that there are 42 specific nanobody sequences. As verified by phage ELISA, 21 sequences were retained, all of which were expressed in form of VHH-Fc fusion protein for purification, and then subject to ELISA. 5% Milk was used as control of non-specific binding, P4 was used as a positive clone (the VH of P4 is shown in SEQ ID NO: 58, and the VL of P4 is shown in SEQ ID NO: 62), and Caplacizumab was used as the negative control. The results show that a total of 12 antibodies including MSLN1, MSLN 2, MSLN 3, MSLN 10, MSLN 11, MSLN 16, MSLN 18, MSLN 27, MSLN 36, MSLN 39, MSLN 41, and MSLN 42 bind MSLN with high specificity and high affinity, and their EC50 is similar to that of the positive antibody P4; and a total of 6 antibodies including MSLN 5, MSLN 6, MSLN 7, MSLN 14, MSLN 29, and MSLN 35 have poor specificity, MSLN 4 and MSLN 15 have very poor specificity, and MSLN 30 does not bind at all.

### Example 2. Identification of Binding Activity of Nanobodies Targeting Three MSLN Domains

### 2.1 Expression and purification of VHH-Fc fusion protein in eukaryotic cells

Specific antibody sequences were selected from the sequencing results, and cloned into the vector pcDNA3.4. The recombinant plasmid was transformed into *E. coli* DH5α (Tiangen Biotech Co., Ltd., Item No. CB101-02), and the bacterial solution was applied evenly onto 2YT solid medium containing 100 µg/mL of ampicillin and left at 37°C overnight. On the next day, a single clone was picked and cultured on a shaking table at 37°C for about 6 h. Half of the bacterial solution was sent for sequencing, and the other half was stored at 4°C. The correctly sequenced clones were amplified and cultured, and plasmids were extracted. The expression plasmids were transfected into EXPI293 by a PEI transfection method, and expressed in a 37°C cell incubator for 5 days. Then, the cell supernatant was collected, and the antibody was purified by ProteinA affinity chromatography column. Finally, an antibody protein with a purity of more than 90% was obtained.

### 2.2 Detection of Specific Binding of VHH-Fc to Human MSLN Protein and Three Domains Thereof by ELISA

A high-absorption 96-well plate was coated with 2 µg/mL of MSLN-His and three MSLN-Domain proteins, and left at 4°C overnight. The plate was washed three times with 1×PBST, blocked with 0.5% BSA at room temperature for 2 h, and washed three times with 1×PBST, into which 100 nM of VHH-Fc was added. The plate was incubated with shaking at room temperature for 1 h, and washed three times with 1×PBST. A secondary antibody Anti-human IgG Fc Antibody HRP (abcam, Item No. ab99759) was added, incubated at room temperature for 30-60 minutes, and washed seven times with 1×PBST. Then, a TMB developer was added, and 2M phosphoric acid was added 3-5 min later to stop the reaction. The absorbance was read at wavelength of 450 nm.

### 2.3. Identification of Binding Activity of Bivalent VHH-Fc to Human MSLN-Transfected 293T and OVCAR3 by Flow Cytometry

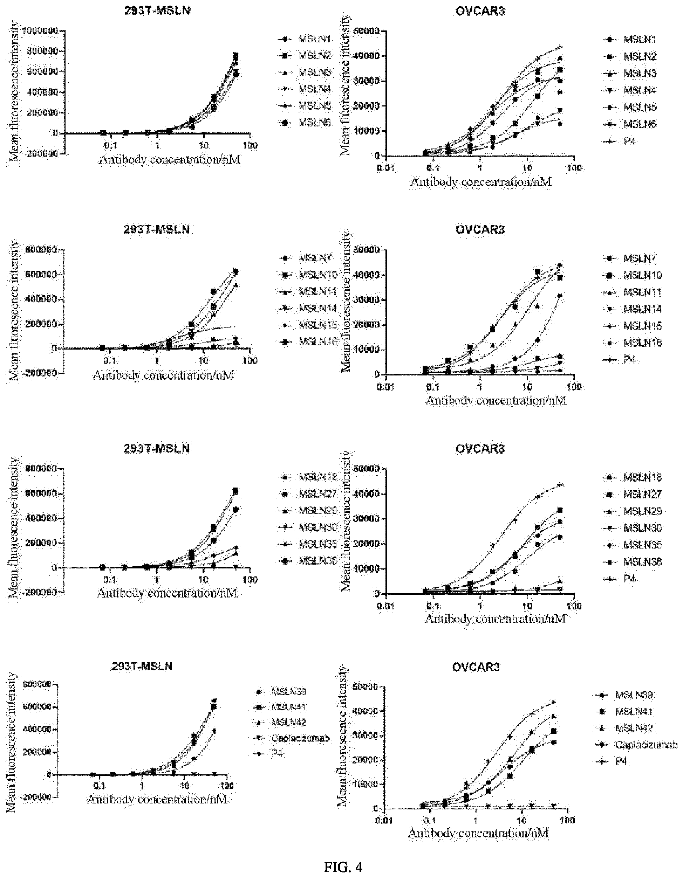

The OVCAR3 and 293T-MSLN cells were resuscitated and passaged. On the day of experiment, the cells were harvested, counted, and adjusted to a cell density of 1×10⁶ cells/ml, with 30 µL per well (3×10⁴ cells/well). The representative MSLN nanobody, positive antibody P4, and negative antibody Caplacizumab were 3-fold diluted to form 7 gradients with 100 nM as the highest concentration, and a PBS control was set, which were added at 30 µL per well, mixed well, and then incubated at 4°C for 1 h. The plate was washed twice with PBS containing 0.1% BSA, and spin-dried at 500g at 4°C for 5 min. The fluorescent secondary antibody Goat pAb to Hu IgG [DyLight 650] (abcam, Item No. ab98593) was diluted at 1:200, added at 30 µL per well, mixed well, and then incubated at 4°C for 30 min. The plate was washed twice with PBS containing 0.1% BSA, spin-dried at 500g at 4°C for 5min, resuspended at 30 µL/well, and loaded on a high-throughput flow cytometer (IQue) for readout. The collected data were used to create a three-parameter fitting curve by Graphpad. The results are shown in FIG. 4. In the drawing, the numbers in the legend represent the sequence numbers corresponding to the MSLN VHH. The results show that MSLN 1, MSLN 2, MSLN 3, MSLN 4, MSLN 5, MSLN 6, MSLN 10, MSLN 11, MSLN 16, MSLN 18, MSLN 27, MSLN 36, MSLN 39, MSLN 41, and MSLN 42 have good flow binding activity to ovarian cancer cell line OVCAR3 with high expression of MSLN and over-expressed cell lines 293-MSLN.

### 2.4. Identification of Non-Specific Binding of Bivalent VHH-Fc to Tissue Cells by Flow Cytometry

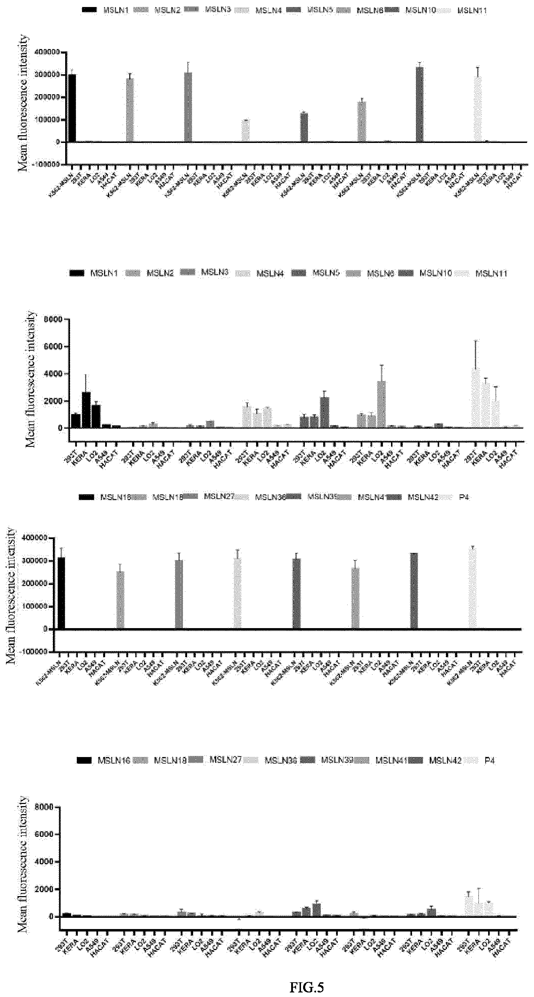

The 293T, LO2, KERA, A549, HACAT, and K562-MSLN cells were resuscitated and passaged. On the day of experiment, the cells were harvested, counted, and adjusted to a cell density of 1×10⁶ cells/ml, with 30 µL per well (3×10⁴ cells/well). The representative MSLN nanobody and positive antibody P4 with concentration of 20 µg/mL were mixed well at 30 µL per well and incubated at 4°C for 1 h. The plate was washed twice with PBS containing 0.1% BSA, and spin-dried at 500g at 4°C for 5 min. The fluorescent secondary antibody Goat pAb to Hu IgG [DyLight 650] (abcam, Item No. ab98593) was diluted at 1:200, added at 30 µL per well, mixed well, and incubated at 4°C for 30 min. The plate was washed twice with PBS containing 0.1% BSA, spin-dried at 500g at 4°C for 5 min, resuspended at 30 µL/well, and loaded on a high-throughput flow cytometer (IQue) for readout. The results are shown in FIG. 5. The results show that the sequences MSLN 1, MSLN 4, MSLN 5, MSLN 6, MSLN 11, MSLN 36, and MSLN 42 all have non-specific binding activity to 293T, LO2, KERA, A549, and HACAT cells.

### Example 3 Detection of CAR-T Lentiviral Packing Kit

### 3.1 Lentiviral packaging

The vector system for constructing the lentiviral plasmid vector of the present application belongs to the third generation of lentiviral vector system, which includes a total of three plasmids: packaging plasmid psPAX2 encoding Gag-Pol protein and Rev protein; PMD2.G plasmid encoding envelope protein VSV-G; and core plasmid encoding the target gene CAR. Based on the BBz/L6 platform, the expression of the coding CAR gene in the core plasmid was regulated by the elongation factor-1α (EF-1α) promoter. The virus was packaged by the following process: 1×10⁶ 293FT cells were suspended in 2 mL of DMEM (10% FBS) medium, and plated on a 6-well plate. The cells were fully dispersed and cultured under 5% CO₂ at 37°C for about 24 h. The plasmids and reagents were equilibrated at room temperature. 100 µL of Opti-MEM medium contained 4.5 µg of (psPAX2:PMD2.G: core plasmid=3:2:4) and 13.5 µL of (plasmid: FuGENEHD = 1:3) transfection reagent (Promega, Item No.: E2311). The packaged plasmids were mixed thoroughly, and the transfection reagent was slowly added dropwise. After addition, they were gently mixed well, and left at room temperature for 15 min. The original 400 µL of medium DMEM (10% FBS) was aspirated, and a liposome cationic complex (100 µL of Opti-MEM+PSH1+PMH2+core plasmid+FuGENEHD) was added dropwise in various regions. The mixture was cultured under 5% CO₂ at 37°C for about 17 h. The plasmid-containing medium (about 200 µL left, One by one) was removed, supplemented with 2.5 mL of DMEM medium containing 5% FBS (preheated at 37°C for 30 mins), and cultured under 5% CO₂ at 37°C for about 24-26 h. The virus supernatant was collected, centrifuged at 3000 rpm for 10 min, and subpackaged into about 720 µL in cryovials for cryopreservation at -80°C. An appropriate amount was additionally taken for lentivirus titer determination.

### 3.2 Titration of Lentivirus Titer

293T cells in good condition were washed with 1×PBS, digested with 1.5 mL of 0.25% trypsin at 37°C, and quenched with 11 mL of medium. The cell system was adjusted to 2×10⁵ cells/well/2.5mL. 10 µg/mL of polybrene was added to the cell line medium at a ratio of 1:1000 and mixed thoroughly. 1 mL of cell suspension was added to each well of a 12-well plate. 5 µL of the resultant virus stock solution was added dropwise in various regions to 2.5 mL for each well and mixed thoroughly. A virus-free cell suspension was set as blank control for flow cytometry. The cells were cultured under 5% CO₂ at 37°C for about 42-48 h. The supernatant was removed. The cells were washed with PBS and digested with 400 µL of 0.25% trypsin (gibco, Item. No.: 25200072) at 37°C for about 1 min. The digestion was terminated with 2.5 mL of complete medium. The cells were gently pipetted with 1 mL pipette until being fully dispersed, which were transferred to a 1.5 mL EP tube and counted. 5×10⁵ cells were taken for flow cytometry.

### Example 4 Infection and Amplification of T Cells

The PBMC cells were resuscitated. 11 mL of X-VIVO was added and centrifuged at 500 g for 5 min. 12 mL of X-VIVO was added and centrifuged at 400 g for 5 min. 12 mL of X-VIVO was added and centrifuged at 300 g for 5 min. The cells were suspended in 1 mL of X-VIVO for counting. The cell density was adjusted to 1×10⁶ cells/ml, and CD3/CD28 (4×10⁸/ml) magnetic beads (Thermo Fisher, Item No.: 11131D) were added at a ratio of 1:3 (cells:magnetic beads) to activate T cells, and mixed thoroughly. 700 µL of cell suspension was added into each well of a 12-well plate (at 7×10⁵ cells/well/700 µl). The activated PBMC were cultured at 37°C in a CO₂ incubator for 18 h. The virus supernatant was added at a MOI of 4-5, and polybrene was added to a final concentration of 5 µg/mL (1:2000, by final volume). Corresponding medium was added for replenishment, and the cells were mixed well. A blank control was set. The cells were centrifuged in a horizontal centrifuge at 1200 rpm for 1 h; cultured at 37°C in a 5% CO₂ incubator for 24 h. The cells were gently mixed well, collected in a 1.5 mL EP tube, and centrifuged at 300g for 5 min. The supernatant was removed, with about 50-100 µL medium left. The cells were resuspended in 1 mL of X-VIVO medium. Additional 500 µL of medium was supplemented into the 12-well plate and the cells were cultured at 37°C in a 5% CO₂ incubator for 48 h. The medium was observed for its color, and an appropriate amount of X-VIVO medium (800 µL-1 mL) was added and cultured at 37°C, 5% CO₂. The cells were counted every 2 days, and adjusted to a cell density of 7×10⁵/ml, a 6-well plate/3 ml. A total of 2.1 × 10⁶ cells were cultured in a 37°C, 5% CO₂ incubator for 24 h, observed and supplemented with an appropriate amount of medium (1 mL) for 24 h. The cells were gently mixed well and counted. The amplification multiple T2 was calculated by dividing the total number of counted cells by the previous total cell number of 2.1E+6. The cell density was adjusted to 7×10⁵/ml, 6-Well-plate/3ml, and the total cell number was 2.1×10⁶ for passage. 6×10⁵ cells were used for detecting the CAR-T positive rate and CD25/CD69 CAR (by removing magnetic beads for 1 min). The cells were cultured in a 37°C, 5% CO₂ incubator for 24 h, observed and supplemented with an appropriate amount of medium (1 mL) for 24 h, and gently mixed well for counting. The amplification multiple T3 was calculated by dividing the total number of counted cells by the previous total cell number of 2.1×10⁶. The cell density was adjusted to 7×10⁵/ml, 6-Well-plate/3ml, and the total cell number was 2.1×10⁶ for passage. 9E5 cells were used for detecting the CAR-T positive rates and PD-L1/TIM3/LAG3 CAR (by removing magnetic beads for 1 min). An appropriate number of cells were used in the *in vitro* tumor killing activity experiment, which were cultured in a 37°C, 5% CO₂ incubator for 24 h, observed and supplemented with an appropriate amount of medium (1 mL-2.5 mL) for 48 h, and gently mixed well for counting. The amplification multiple T4 was calculated by dividing the total number of counted cells by the previous total cell number of 2.1 × 10⁶. Corresponding cells were used for CAR-T repeated stimulation experiment. An appropriate number of cells were used for detecting the CAR-T positivity rate, CD25/CD69, and PD-1/TIM3/LAG3 CAR (by removing magnetic beads for 1 min). The amplification multiple was calculated as T1*T2*T3*T4.

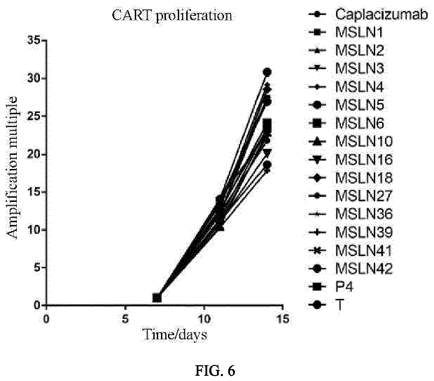

The results are shown in FIG. 6. In the drawing, the numbers in the legend represent the sequence numbers corresponding to the MSLN VHH. MSLN3 (with VHH sequence as set forth in SEQ ID NO: 13), MSLN10 (with VHH sequence as set forth in SEQ ID NO: 18), MSLN16 (with VHH sequence as set forth in SEQ ID NO: 8), MSLN27 (with VHH sequence as set forth in SEQ ID NO: 23), MSLN36 (with VHH sequence as set forth in SEQ ID NO: 28), MSLN41 (with VHH sequence as set forth in SEQ ID NO: 33), MSLN42 (with VHH sequence as set forth in SEQ ID NO: 38) were selected, wherein MSLN0 was the negative control Caplacizumab, P4 was the positive control (with VH sequence as set forth in SEQ ID NO: 58, and VL sequence as set forth in SEQ ID NO: 62), and T was the blank control without target cells. The results show that all antibodies had similar proliferation rate to that of the positive antibodies.

### Example 5 Detection of In Vitro Tumor Killing Activity of CAR-T Cells

### CytoTox 96^{®} Non-Radioactive Cytotoxicity Assay

LDH is a stable cytoplasmic enzyme that is released during cell lysis. The release mode is substantially the same as the release mode of 51Cr in radioactive analysis. The released LDH is in the culture supernatant and can be detected by the coupling enzyme reaction. In the enzyme reaction, LDH can convert a tetrazolium salt (INT) into red formazan, and the amount of the red product generated is proportional to the number of lysed cells (Promega, Item No.: G1780). To detect the CAR-T positive rate, the cells were adjusted with uninfected blank T cells to allow that the proportion of infected CAR was consistent between groups and the total number of cells was consistent in various groups. The number of target cells was 2×10⁴/96-well-plate/well, and the effector-to-target ratio (E :T) = 3:1, 1:1, 1:3, 1:9. A well containing only equivalent amount of effector cells as the experimental group was set as the effector cell self-releasing LDH background group; a well containing only equivalent amount of blank T cells was set as the T cell self-releasing LDH blank control group; a well containing only blank T cells at the same effector-to-target ratio as the experimental group was set as the T + target cell negative control group; a well containing only equivalent amount of target cells as the experimental group was set as the target cell self-releasing LDH background group; a well containing only equivalent amount of target cells as the experimental group was set as the target cell maximum release LDH background group; an independent well containing only 200 µL of cell medium was set as the medium background LDH control well, and an independent well containing only 200 µL of cell medium was set as the volume correction control well. Corresponding effector cells and target cells were co-incubated in 200 µl of X-VIVO medium (without FBS), cultured in a 37°C, 5% CO₂ incubator for 20-24 h. The cells were digested, counted, and corresponding amount of target cells were resuspended in 100 µl of X-VIVO, and plated in a 96-well plate. The corresponding amount of effector cells were taken according to the effector-to-target ratio, and supplemented with corresponding blank T cells at 500g for 5 min. The cells were resuspended in 100 µL of X-VIVO and mixed with target cells in the 96-well plate, and cultured under 5% CO₂ at 37 °C for 20-24 h. About 45 min in advance, 20 µL of lysis solution (10X) was added into the volume correction control well and the target cell maximum release LDH background well, and cultured at 37°C in a 5% CO₂ incubator for 45 min, and centrifuged at 250×g for 4 min. 50 µl of the supernatant was transferred into the enzyme analysis plate, and the formulated substrate (with a detection buffer) was added at 50 µL/well for incubation at room temperature in the dark for 20-30 min. A stop solution was added at 50 µL/well. The color was observed (to avoid bubbles), and the OD490 values were measured.

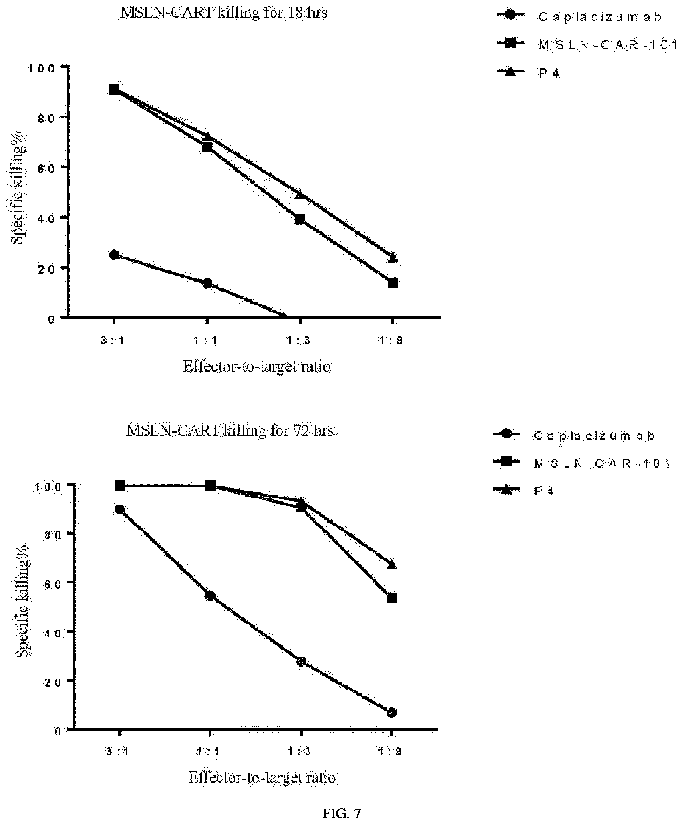

1×10⁴ tumor cells SK-OV3-Luciferase-GFP were co-cultured with exemplary MSLN-CAR-T cells at different effector-to-target ratios for 18 h and 72 h, and then detected for the killing effect of CAR-T cells on tumor cells. The results are shown in FIG. 7, indicating that MSLN-CAR-101 (in which the amino acid sequence of the targeting moiety of the chimeric antigen receptor is as set forth in SEQ ID NO: 13, the amino acid sequence of the transmembrane region is as set forth in SEQ ID NO: 40, the amino acid sequence of the costimulatory domain is as set forth in SEQ ID NO: 39, the amino acid sequence of the intracellular signaling domain is as set forth in SEQ ID NO: 41, the amino acid sequence of the hinge region is as set forth in SEQ ID NO: 42, and the MSLN-CAR-101 cells further expresses a foreign protein fragment as set forth in SEQ ID NO: 44) has a killing effect similar to that of the positive sequence P4.

### Example 6 Detection of Cytokines

The CAR-T was prepared according to the infection and amplification method of T cells in Example 4. An appropriate amount of supernatant was co-incubated at E:T=1:1 for detection of IL-2, IFN-γ and other cytokines. To detect the CART positive rate, the cells were adjusted with uninfected blank T cells to allow that the proportion of infected CAR was consistent between groups and the total number of cells was consistent in various groups (the desired CAR-T cell number, with the minimum CAR positive proportion as the baseline); and the total number of T cells was consistent; VT (T + CELL group) = CART + T. A well containing an equivalent amount of blank T cells as the experimental group was set as the separate T cell group; and a well containing an equivalent amount of effector cells as the experimental group was set as the separate effector cell group. The number of target cells was 1×10⁴/96-well/well, and the effector:target cells (E:T) = 1:1. The cells were co-incubated in accordance with a corresponding effector-to-target ratio of 1:1, the effector cells (containing the CAR-T positive cell 1E4) were mixed with the target cells (1E4), supplemented with serum-free 1 X-VIVO medium to a final volume of 200 µL, gently mixed well at 37°C in a 5% CO₂ incubator for 24 h. The cells were centrifuged at 400×g for 5 min. 50 µL of cell supernatant was used for cytokine detection.

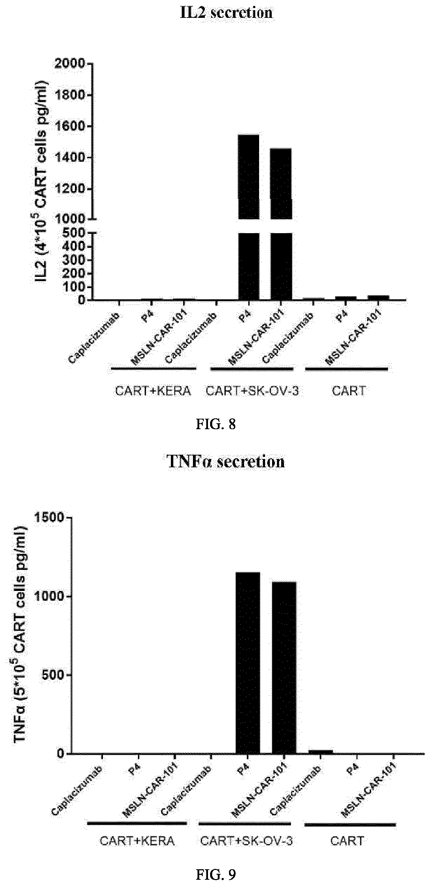

The results are shown in FIGs. 8-10, in which KERA represents MSLN-negative cells and SK-OV3 represents MSLN-positive cells. When the CAR-T cells were co-cultured with SK-OV3, the CAR-T cells were activated to produce a large amount of cytokines IL-2, TNF-α and IFN-y. Among them, MSLN-CAR-101 and the positive sequence P4 have equivalent amount of cytokine release. In addition, when the CAR-T cells were co-cultured with KERA, no CAR-T cells could be activated, and no cytokines were released.

### Example 7 Animal Pharmaceutical Efficacy Test in NSG Mice

Mice were subcutaneously injected with 3×10⁶ SKOV3 tumor cell lines. Two weeks later, the tumor size reached 100-200 mm³, and then the mice were randomly divided into groups. On the next day, the mice received intravenous refusion of CAR-T at a cell amount of 5×10⁶. The tumors were measured three times a week, and the test was observed until Day 47. Finally, a tumor growth curve was plotted by Graphpad. Among them, 5E6 represents ovarian cancer cells inoculated with 5*10⁶ SK-OV3.

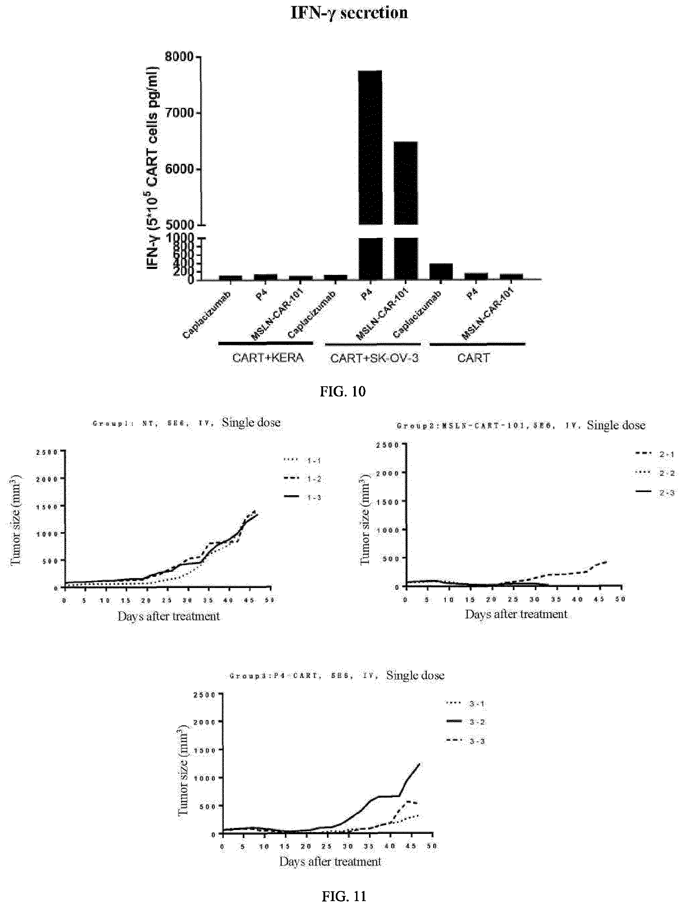

The results are shown in FIG. 11, showing that MSLN-CAR-101 significantly inhibited the tumor growth.

The foregoing detailed description is provided by way of explanation and examples and is not intended to limit the scope of the appended claims. Various changes of the embodiments currently set forth in this application are obvious to those of ordinary skills in the art and are reserved within the scope of the appended claims and their equivalents.

## Claims

1. An isolated antigen binding protein, capable of specifically binding to mesothelin (MSLN), wherein said isolated antigen binding protein comprises at least one CDR in an antibody heavy chain variable region VH, said VH comprises an amino acid sequence as set forth in any one of SEQ ID NO: 8, SEQ ID NO: 13, SEQ ID NO: 69, and SEQ ID NO: 70.

2. The antigen binding protein of claim 1, comprising an HCDR3, wherein said HCDR3 comprises an amino acid sequence as set forth in any one of SEQ ID NO: 1, SEQ ID NO: 9, SEQ ID NO: 65, and SEQ ID NO: 66.

3. The antigen binding protein of claim 2, wherein said HCDR3 comprises an amino acid sequence as set forth in any one of SEQ ID NO: 1, SEQ ID NO: 9, SEQ ID NO: 14, SEQ ID NO: 19, SEQ ID NO: 24, SEQ ID NO: 29, and SEQ ID NO: 34.

4. The antigen binding protein of any one of claims 1-3, comprising an HCDR2, wherein said HCDR2 comprises an amino acid sequence as set forth in SEQ ID NO: 67.

5. The antigen binding protein of claim 4, wherein said HCDR2 comprises an amino acid sequence as set forth in any one of SEQ ID NO: 2, SEQ ID NO: 10, SEQ ID NO: 15, SEQ ID NO: 20, SEQ ID NO: 25, SEQ ID NO: 30, and SEQ ID NO: 35.

6. The antigen binding protein of any one of claims 1-5, comprising an HCDR1, wherein said HCDR1 comprises an amino acid sequence as set forth in SEQ ID NO: 68 (X₁X₂X₃MG, wherein X₁ is N, R, S, T or Y, X₂ is N or Y, X₃ is A, N or V).

7. The antigen binding protein of claim 6, wherein said HCDR1 comprises an amino acid sequence as set forth in any one of SEQ ID NO: 3, SEQ ID NO: 11, SEQ ID NO: 16, SEQ ID NO: 21, SEQ ID NO: 26, SEQ ID NO: 31, and SEQ ID NO: 36.

8. The antigen binding protein of any one of claims 1-7, comprising an HCDR1, an HCDR2, and an HCDR3, wherein said HCDR1, HCDR2, and HCDR3 comprises any one group of amino acid sequences selected from:
(1) said HCDR1 comprises an amino acid sequence as set forth in SEQ ID NO: 3, said HCDR2 comprises an amino acid sequence as set forth in SEQ ID NO: 2, and said HCDR3 comprises an amino acid sequence as set forth in SEQ ID NO: 1;
(2) said HCDR1 comprises an amino acid sequence as set forth in SEQ ID NO: 11, said HCDR2 comprises an amino acid sequence as set forth in SEQ ID NO: 10, and said HCDR3 comprises an amino acid sequence as set forth in SEQ ID NO: 9;
(3) said HCDR1 comprises an amino acid sequence as set forth in SEQ ID NO: 16, said HCDR2 comprises an amino acid sequence as set forth in SEQ ID NO: 15, and said HCDR3 comprises an amino acid sequence as set forth in SEQ ID NO: 14;
(4) said HCDR1 comprises an amino acid sequence as set forth in SEQ ID NO: 21, said HCDR2 comprises an amino acid sequence as set forth in SEQ ID NO: 20, and said HCDR3 comprises an amino acid sequence as set forth in SEQ ID NO: 19;
(5) said HCDR1 comprises an amino acid sequence as set forth in SEQ ID NO: 26, said HCDR2 comprises an amino acid sequence as set forth in SEQ ID NO: 25, and said HCDR3 comprises an amino acid sequence as set forth in SEQ ID NO: 24;
(6) said HCDR1 comprises an amino acid sequence as set forth in SEQ ID NO: 31, said HCDR2 comprises an amino acid sequence as set forth in SEQ ID NO: 30, and said HCDR3 comprises an amino acid sequence as set forth in SEQ ID NO: 29; and
(7) said HCDR1 comprises an amino acid sequence as set forth in SEQ ID NO: 36, said HCDR2 comprises an amino acid sequence as set forth in SEQ ID NO: 35, and said HCDR3 comprises an amino acid sequence as set forth in SEQ ID NO: 34.

9. The antigen binding protein of any one of claims 1-8, comprising an H-FR1, wherein a C-terminal of said H-FR1 is connected to an N-terminal of said HCDR1 directly or indirectly, and said H-FR1 comprises an amino acid sequence as set forth in any one of SEQ ID NO: 4, SEQ ID NO: 12, SEQ ID NO: 17, SEQ ID NO: 22, SEQ ID NO: 27, SEQ ID NO: 32, and SEQ ID NO: 37.

10. The antigen binding protein of any one of claims 1-9, comprising an H-FR2, wherein said H-FR2 is located between said HCDR1 and said HCDR2, and said H-FR2 comprises an amino acid sequence as set forth in SEQ ID NO: 5.

11. The antigen binding protein of any one of claims 1-10, comprising an H-FR3, wherein said H-FR3 is located between said HCDR2 and said HCDR3, and said H-FR3 comprises an amino acid sequence as set forth in SEQ ID NO: 6.

12. The antigen binding protein of any one of claims 1-11, comprising an H-FR4, wherein an N-terminal of said H-FR4 is connected to a C-terminal of said HCDR3 directly or indirectly, and said H-FR4 comprises an amino acid sequence as set forth in SEQ ID NO: 7.

13. The antigen binding protein of any one of claims 1-12, comprising an antibody heavy chain variable region VH, wherein said VH comprises an amino acid sequence as set forth in any one of SEQ ID NO: 8, SEQ ID NO: 13, SEQ ID NO: 69, and SEQ ID NO: 70.

14. The antigen binding protein of any one of claims 1-13, comprising an antibody heavy chain variable region VH, wherein said VH comprises an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

15. The antigen binding protein of any one of claims 1-14, comprising an antibody or an antigen binding fragment thereof.

16. The antigen binding protein of claim 15, wherein said antigen binding fragment comprises Fab, Fab', F(ab)₂, an Fv fragment, F(ab')₂, scFv, di-scFv, VHH, and/or dAb.

17. The antigen binding protein of any one of claims 15-16, wherein said antibody is selected from a group consisting of: a monoclonal antibody, a chimeric antibody, a humanized antibody, and a fully human antibody.

18. The antigen binding protein of any one of claims 15-17, wherein said antigen binding fragment is a VHH, and said VHH comprises an amino acid sequence as set forth in any one of SEQ ID NO: 8, SEQ ID NO: 13, SEQ ID NO: 69, and SEQ ID NO: 70.

19. The antigen binding protein of claim 18, wherein said VHH comprises an amino acid sequence as set forth in any one of SEQ ID NO: 8, SEQ ID NO: 13, SEQ ID NO: 18, SEQ ID NO: 23, SEQ ID NO: 28, SEQ ID NO: 33, and SEQ ID NO: 38.

20. The antigen binding protein of any one of claims 1-19, capable of competing with a reference antibody for binding to MSLN (mesothelin) protein, wherein said reference antibody comprises an antibody heavy chain variable region VH, the VH of said reference antibody comprises an HCDR1, an HCDR2, and an HCDR3, and said reference antibody comprises any one group of amino acid sequences selected from:
(1) said HCDR1 comprises an amino acid sequence as set forth in SEQ ID NO: 3, said HCDR2 comprises an amino acid sequence as set forth in SEQ ID NO: 2, and said HCDR3 comprises an amino acid sequence as set forth in SEQ ID NO: 1;
(2) said HCDR1 comprises an amino acid sequence as set forth in SEQ ID NO: 11, said HCDR2 comprises an amino acid sequence as set forth in SEQ ID NO: 10, and said HCDR3 comprises an amino acid sequence as set forth in SEQ ID NO: 9;
(3) said HCDR1 comprises an amino acid sequence as set forth in SEQ ID NO: 16, said HCDR2 comprises an amino acid sequence as set forth in SEQ ID NO: 15, and said HCDR3 comprises an amino acid sequence as set forth in SEQ ID NO: 14;
(4) said HCDR1 comprises an amino acid sequence as set forth in SEQ ID NO: 21, said HCDR2 comprises an amino acid sequence as set forth in SEQ ID NO: 20, and said HCDR3 comprises an amino acid sequence as set forth in SEQ ID NO: 19;
(5) said HCDR1 comprises an amino acid sequence as set forth in SEQ ID NO: 26, said HCDR2 comprises an amino acid sequence as set forth in SEQ ID NO: 25, and said HCDR3 comprises an amino acid sequence as set forth in SEQ ID NO: 24;
(6) said HCDR1 comprises an amino acid sequence as set forth in SEQ ID NO: 31, said HCDR2 comprises an amino acid sequence as set forth in SEQ ID NO: 30, and said HCDR3 comprises an amino acid sequence as set forth in SEQ ID NO: 29; and
(7) said HCDR1 comprises an amino acid sequence as set forth in SEQ ID NO: 36, said HCDR2 comprises an amino acid sequence as set forth in SEQ ID NO: 35, and said HCDR3 comprises an amino acid sequence as set forth in SEQ ID NO: 34.

21. The antigen binding protein of any one of claims 1-20, comprising an antibody heavy chain constant region, wherein said antibody heavy chain constant region is derived from IgG.

22. The antigen binding protein of any one of claims 1-21, comprising an antibody heavy chain constant region, wherein said antibody heavy chain constant region is derived from human IgG.

23. The antigen binding protein of any one of claims 1-22, comprising an antibody heavy chain constant region, wherein said antibody heavy chain constant region is derived from human IgG1.

24. The antigen binding protein of any one of claims 21-23, wherein said antibody heavy chain constant region comprises an Fc region of IgG.

25. The antigen binding protein of claim 24, wherein said Fc region comprises an amino acid sequence as set forth in SEQ ID NO: 54.

26. A chimeric antigen receptor, comprising a targeting moiety, wherein said targeting moiety comprises the antigen binding protein of any one of claims 1-25.

27. The chimeric antigen receptor of claim 26, comprising costimulatory domain, wherein said costimulatory domain comprises a costimulatory domain derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

28. The chimeric antigen receptor of claim 27, wherein said costimulatory domain is an intracellular costimulatory signaling region derived from 4-1BB.

29. The chimeric antigen receptor of any one of claims 27-28, wherein said costimulatory domain comprises an amino acid sequence as set forth in SEQ ID NO: 39.

30. The chimeric antigen receptor of any one of claims 26-29, comprising an intracellular signaling domain, wherein said intracellular signaling domain comprises an intracellular signaling domain derived from one or more proteins selected from a group consisting of CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP-12, and a domain containing at least one ITAM.

31. The chimeric antigen receptor of claim 30, wherein said intracellular signaling domain is a signaling domain derived from CD3ζ.

32. The chimeric antigen receptor of claim 31, wherein said intracellular signaling domain comprises an amino acid sequence as set forth in SEQ ID NO: 41.

33. The chimeric antigen receptor of any one of claims 26-32, comprising a transmembrane region, wherein said transmembrane region comprises a transmembrane domain derived from one or more proteins selected from a group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

34. The chimeric antigen receptor of claim 33, wherein said transmembrane region is a transmembrane region derived from CD8.

35. The chimeric antigen receptor of claim 34, wherein said transmembrane region comprises an amino acid sequence as set forth in SEQ ID NO: 40.

36. The chimeric antigen receptor of any one of claims 26-35, comprises a hinge region between the targeting moiety and the transmembrane region, wherein said hinge region comprises a hinge region derived from one or more proteins selected from a group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

37. The chimeric antigen receptor of claim 36, wherein said hinge region is a hinge region derived from CD8.

38. The chimeric antigen receptor of claim 37, wherein said hinge region comprises an amino acid sequence as set forth in SEQ ID NO: 42.

39. The chimeric antigen receptor of any one of claims 26-38, further comprising a signal peptide.

40. The chimeric antigen receptor of claim 39, wherein said signal peptide is derived from a signal peptide of CD8 protein.

41. The chimeric antigen receptor of claim 40, wherein said signal peptide comprises an amino acid sequence as set forth in SEQ ID NO: 43.

42. The chimeric antigen receptor of any one of claims 26-41, further comprising a low-density lipoprotein receptor-related protein or a fragment thereof.

43. The chimeric antigen receptor of claim 42, wherein said low-density lipoprotein receptor-related protein or the fragment thereof comprises one or more selected from a group consisting of low-density lipoprotein receptor-related proteins 1-12 and functional fragments thereof.

44. The chimeric antigen receptor of any one of claims 42-43, wherein said low-density lipoprotein receptor-related protein or the fragment thereof is low-density lipoprotein receptor-related proteins 5 and/or 6 or fragments thereof.

45. The chimeric antigen receptor of any one of claims 42-44, wherein said low-density lipoprotein receptor-related protein or the fragment thereof comprises an amino acid sequence as set forth in SEQ ID NO: 44.

46. The chimeric antigen receptor of any one of claims 26-45, comprising an amino acid sequence as set forth in any one of SEQ ID NO: 47, SEQ ID NO: 48, SEQ ID NO: 49, SEQ ID NO: 50, SEQ ID NO: 51, SEQ ID NO: 52, and SEQ ID NO: 53.

47. A polypeptide, comprising the antigen binding protein of any one of claims 1-25.

48. One or more isolated nucleic acid molecules, encoding the antigen binding protein of any one of claims 1-25, and/or the chimeric antigen receptor of any one of claims 26-46.

49. The nucleic acid molecules of claim 48, further comprising a promoter.

50. The nucleic acid molecules of claim 49, wherein said promoter is a constitutive promoter.

51. The nucleic acid molecules of any one of claims 49-50, wherein said promoter is an EF1α promoter.

52. A vector, comprising the nucleic acid molecules of any one of claims 48-51.

53. The vector of claim 52, comprising a viral vector.

54. The vector of any one of claims 52-53, comprising a lentiviral vector.

55. A cell, comprising the antigen binding protein of any one of claims 1-25, the chimeric antigen receptor of any one of claims 26-46, the polypeptide of claim 47, the nucleic acid molecules of any one of claims 48-51, and/or the vector of any one of claims 52-54.

56. The cell of claim 55, which is an immune effector cell.

57. The cell of any one of claims 55-56, comprising T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

58. The cell of any one of claims 55-57, which is a T cell.

59. The cell of any one of claims 55-58, further comprising and/or expressing a low-density lipoprotein receptor-related protein or a fragment thereof.

60. The cell of claim 59, wherein said low-density lipoprotein receptor-related protein or the fragment thereof comprises one or more selected from a group consisting of low-density lipoprotein receptor-related proteins 1-12 and functional fragments thereof.

61. The cell of any one of claims 59-60, wherein said low-density lipoprotein receptor-related protein or the fragment thereof is low-density lipoprotein receptor-related proteins 5 and/or 6 or fragments thereof.

62. The cell of any one of claims 59-61, wherein said low-density lipoprotein receptor-related protein or the fragment thereof comprises an amino acid sequence as set forth in SEQ ID NO: 44.

63. A method for preparing the antigen binding protein of any one of claims 1-25 and/or the chimeric antigen receptor of any one of claims 26-46, comprising culturing the cell of any one of claims 51-58 under a condition enabling the expression of the antigen binding protein of any one of claims 1-25 and/or the chimeric antigen receptor of any one of claims 26-46.

64. A method for preparing a modified immune effector cell, comprising introducing the vector of any one of claims 52-54 into said immune effector cell.

65. A pharmaceutical composition, comprising the isolated antigen binding protein of any one of claims 1-25, the chimeric antigen receptor of any one of claims 26-46, the polypeptide of claim 47, the nucleic acid molecules of any one of claims 48-51, the vector of any one of claims 52-54, and/or the cell of any one of claims 55-62, as well as optionally a pharmaceutically acceptable carrier.

66. Use of the isolated antigen binding protein of any one of claims 1-25, the chimeric antigen receptor of any one of claims 26-46, the polypeptide of claim 47, the nucleic acid molecules of any one of claims 48-51, the vector of any one of claims 52-54, the cell of any one of claims 55-62, and/or the pharmaceutical composition of claim 65 in the preparation of a drug for preventing, treating and/or alleviating a disease or a disorder associated with abnormal expression of MSLN.

67. The use of claim 66, wherein said disease or disorder associated with abnormal expression of MSLN comprises a tumor.

68. The use of claim 67, wherein said tumor comprises a solid tumor.

69. The use of claim 67, wherein said tumor comprises a non-solid tumor.

70. The use of any one of claims 67-69, wherein said tumor comprises an MSLN antigen-expressing tumor.

71. The use of any one of claims 67-70, wherein said tumor comprises ovarian cancer, pancreatic cancer, gastric cancer, mesothelioma, bile duct cancer, triple-negative breast cancer, and/or endometrial cancer.

72. A method for preventing, treating and/or alleviating a disease or a disorder associated with abnormal expression of MSLN, comprising administering to a subject in need thereof the antigen binding protein of any one of claims 1-25, the chimeric antigen receptor of any one of claims 26-46, the polypeptide of claim 47, the nucleic acid molecules of any one of claims 48-51, the vector of any one of claims 52-54, the cell of any one of claims 55-62, and/or the pharmaceutical composition of claim 65.

73. The method of claim 72, wherein said disease or disorder associated with abnormal expression of MSLN comprises a tumor.

74. The method of claim 73, wherein said tumor comprises a solid tumor.

75. The method of claim 73, wherein said tumor comprises a non-solid tumor.

76. The method of any one of claims 73-75, wherein said tumor comprises an MSLN antigen-expressing tumor.

77. The method of any one of claims 73-76, wherein said tumor comprises ovarian cancer, pancreatic cancer, gastric cancer, mesothelioma, bile duct cancer, triple-negative breast cancer, and/or endometrial cancer.
